# EUROPEAN PATENT SPECIFICATION

(11) **EP 1 322 622 B1**
(45) Date of publication and mention of the grant of the patent: **31.10.2012**
(21) Application number: 01985252.4
(22) Date of filing: 08.09.2001
(51) Int. Cl.: C07D 235/26, A61K 31/4184, A61P 43/00, C07D 401/12, C07D 403/12, C07D 471/04, C07D 401/14, C07D 401/06, C07D 471/10, C07D 405/12

(54) **NEW N, N'-DISUBSTITUTED BENZIMIDAZOLONE DERIVATIVES WITH AFFINITY AT THE SEROTONIN AND DOPAMINE RECEPTORS**
NEUE N,N'-DISUBSTITUIERTE BENZIMIDAZOLDERIVATE MIT AFFINITÄT AN DEN SEROTONIN- UND DOPAMINREZEPTOREN
DERIVES BENZIMIDAZOLONE N,N'-DISUBSTITUES PRESENTANT UNE AFFINITE POUR LES RECEPTEURS DE LA SEROTONINE ET DE LA DOPAMINE

(30) Priority: 19.09.2000 EP 00830624
(43) Date of publication of application: 02.07.2003
(73) Proprietor: Sprout Pharmaceuticals, Inc., Raleigh, NC 27609 (US)
(72) Inventor: CEREDA, Enzo, I-15067 Novi Ligure (IT); MAIOCCHI, Luciano, I-22012 Cernobbio (IT); BRAMBILLA, Alessandro, I-20136 Milano (IT); GIRALDO, Ettore, I-20137 MIlano (IT); MONFERINI, Eugenia, I-20124 Milano (IT); SCHIAVI, Giovanni, Battista, I-46041 Asola MN (IT)
(74) Representative: Hally, Anna-Louise
(86) International application number: PCT/EP2001/010376
(87) International publication number: WO 2002/024661

(56) References cited:
- EP-A- 0 816 356
- US-A- 4 200 641
- US-A- 5 576 318
- US-A- 5 883 094
- DATABASE CA [Online] CHEMICAL ABSTRACTS SERVICE, COLUMBUS, OHIO, US; COLLINO, F. ET AL: "Mannich bases of benzimidazoles, benzotriazoles and other analogous compounds, with pharmacological activity. II" retrieved from STN Database accession no. 98:16650 XP002197885 & BOLL. CHIM. FARM. (1982), 121(5), 221-9 ,

## Description

The present invention relates to novel pharmacologically active N,N'-disubstituted benzimidazolone derivatives and their addition salts which bind the serotonin or dopamine receptors, to their preparation and their use for therapeutic purposes. These compounds are able to discriminate the different serotonin and dopamine receptor subtypes like 5-HT_{1A}, 5-HT_{2A} and D₄ at which they can act as agonists or antagonists. Owing to this pharmacological activity, the present compounds are useful in the treatment of anxiety disorders, affective disorders such as depression, psychosis and schizophrenia, eating disorders, sexual disorders, Parkinson, stroke and traumatic brain injury.

### Background of the invention

Serotonin (5-HT) and dopamine (DA) recognise several well defined cell surface receptor subtypes. Among these, 5-HT_{1A} and 5-HT_{2A} having a high and a low affinity for 5-HT, respectively, and D₄ at which DA has high affinity, have been implicated in many Central Nervous System disorders.

In the previous art, several classes of compounds able to interfere with the neurotransmission at 5-HT or DA receptor subtypes are known. Particularly, derivatives based on the core structure of the aryl piperazine and benzimidazolone have been described (e.g. GB 2023594, U.S. Pat. 3,472,854, U.S. Pat. 4,954,503, WO-9616949, WO-9501965 and WO-9833784), and targeted both to generic 5-HT or DA receptors and to a specific receptor subtype. In another patent (U.S. Pat. 5,576,318) are described compounds based both on the benzimidazolone and phenylpiperazine structures: in this latter case the described affinities are limited to 5-HT_{1A} and 5-HT_{2A} receptor subtypes.

Benzimidazolone derivatives which have affinity for the D4 dopamine receptor have been disclosed in U.S. Pat. 5,883,094.

### Detailed description of the invention

Now we describe, and this is the object of the present invention, new derivatives of a benzimidazolone core structure. The N-substituents are alkyl chains bearing additional hydrophilic functional groups whereas the N'-substituents are alkyl or alkenyl spacers connecting the benzimidazolone scaffold to a large set of secondary amines bearing other diversity points. The compounds included in this invention possess an interesting affinity profile at the said serotonin and dopamine receptor subtypes: indeed some of them have a high and prefential affinity at a given site (e.g. 5-HT_{1A}, 5-HT_{2A}, or D₄) whereas some others have a mixed affinity at the said receptors. Moreover a selected pool of compounds possesses an agonistic activity at the 5-HT_{1A} receptor coupled with an antagonistic activity at the 5-HT_{2A} receptor.

Owing to their peculiar profile, the present compounds may play a role in the regulation of neurotransmission at the serotonin and/or the dopamine sites and thus may be of value in the treatment of those diseases where an altered functioning of neurosignal transmission is present. Examples of these disorders include anxiety, depression, schizophrenia, Parkinson, sleep, sexual and eating disorders, stroke and brain injury. Particularly the compounds included in the present invention can be of value in the treatment of depression according to the mounting evidence that 5-HT_{1A} full agonists or high efficiency partial agonists are required for a robust antidepressant effect. In fact, electro physiology studies suggest that repeated administration of a variety of antidepressant treatments facilitate 5-HT_{1A} neurotransmission in the hippocampus, possibly through either an increased sensitivity of post-synaptic 5-HT_{1A} receptors or a decreased sensitivity of 5-HT_{1A} autoreceptors. Furthermore, there is some evidence from controlled clinical trials to support this suggestion. In addition the compound's ability to block the 5-HT_{2A} receptor is also of value: indeed, the stimulation of 5-HT_{1A} and 5-HT_{2A} receptors lead to opposite electrical events, inhibitory and excitatory, respectively. Thus only a concurrent activation of 5-HT_{1A} coupled with antagonism at 5-HT_{2A} receptors may completely and rapidly inhibit 5-HT post-synaptic cells, an important physiological event for antidepressant effects.

The present invention discloses compounds of general formula (I) 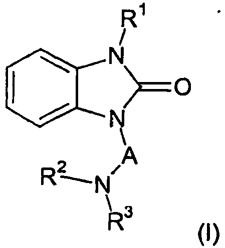 wherein
- R¹: denotes C₁-C₆-alkyl, preferably C₁-C₄-alkyl, being substituted by a group selected from OH, C₁-C₆-alkoxy, -OCONHC₁-C₆-alkyl, -OCONHC₁-C₆-alkyl, -NHSO₂C₁-C₆-alkyl and -NHCOC₁-C₆-alkyl, or
- R¹: denotes C₁-C₆-alkyl, preferably C₁-C₄-alkyl, being substituted by a saturated or unsaturated 5- or 6-membered heterocycle containing one or two heteroatoms selected from the group consisting of nitrogen and oxygen, said heterocycle being optionally substituted by a group selected from C₁-C₄-alkyl, halogen and benzyl;
- R² and R³: together with the nitrogen form a saturated or unsaturated 5- or 6-membered heterocyclic ring which may contain nitrogen or oxygen as an additional heteroatom, whilst the heterocyclic ring is substituted by a group selected from phenyl, benzyl and diphenylmethyl, said group being optionally mono- or di-substituted by one or two groups selected from CF₃, C₁-C₄-alkyl, C₁-C₄-alkoxy, phenyl, benzyl, halogen and OH, or
- R² and R³: together with the nitrogen form a saturated or unsaturated 5- or 6-membered heterocyclic ring which may contain nitrogen or oxygen as an additional heteroatom, said heterocyclic ring being linked via a single bond, a methylene-bridge or spiro-connected to another saturated or unsaturated heterocyclic group containing one or two heteroatoms selected from oxygen and nitrogen, said heterocyclic group being optionally mono- or di-substituted by a group selected from CF₃, C₁-C₄-alkyl, C₁-C₄-alkoxy, phenyl, benzyl, halogen, =O and OH, or
- R² and R³: together with the nitrogen form a saturated or unsaturated bi- or tricyclic heterocyclic ring-system which may contain nitrogen or oxygen as an additional heteroatom, said heterocyclic ring-system being optionally substituted by a group selected from CF₃, C₁-C₄-alkyl, C₁-C₄-alkoxy, phenyl, benzyl, halogen, =O and OH;
- A: denotes C₁-C₆-alkylen, preferably C₁-C₄-alkylen, C₂-C₆-alkenylen, preferably C₂-C₄-alkenylen, or C₂-C₆-alkynylen, preferably C₂-C₄-alkynylen,
or a pharmaceutically acceptable salt thereof.

Preferred compounds are those of formula (I), wherein
- R¹: denotes C₁-C₄-alkyl, preferably C₂-C₃-alkyl, being substituted by a group selected from OH, C₁-C₄-alkoxy, -OCONHC₁-C₄-alkyl, -OCONHC₁-C₄-alkyl, -NHSO₂C₁-C₄-alkyl and -NHCOC₁-C₄-alkyl, or
- R¹: denotes C₁-C₄-alkyl, preferably C₂-C₃-alkyl, being substituted by a saturated or unsaturated 6-membered heterocycle containing one or two heteroatoms selected from the group consisting of nitrogen and oxygen;
- R² and R³: together with the nitrogen form a saturated or unsaturated 5- or 6-membered heterocyclic ring which may contain nitrogen as an additional heteroatom, whilst the heterocyclic ring is substituted by a group selected from phenyl, benzyl, diphenylmethyl, pyridinyl, pyrimidinyl, benzimidazolonyl and 3,4-methylendioxibenzyl, said group being optionally mono- or di-substituted by a group selected from CF₃, C₁-C₄-alkyl, C₁-C₄-alkoxy, halogen and OH;
- A: denotes C₁-C₄-alkylen or C₂-C₄-alkenylen,
or a pharmaceutically acceptable salt thereof.

Also preferred compounds are those of formula (I), wherein
- R¹: denotes ethyl, being substituted by a group selected from OH, OCH₃, OCH₂CH₃, -OCONHCH₃, -OCONHCH₂CH₃, -NHSO₂CH₃, -NHSO₂CH₂CH₃, - NHCOCH₃, -NHCOCH₂CH₃, morpholinyl, piperazinyl and piperidinyl
- R² and R³: together with the nitrogen form a 6 membered saturated or unsaturated heterocyclic ring which may contain nitrogen as an additional heteroatom, whilst the heterocyclic ring is substituted by a group selected from phenyl, pyridinyl, pyrimidinyl, benzimidizalonyl, and substituted phenyl being mono- or di-substituted by a group selected from CF₃, CH₃, OCH₃, F and Cl;
- A: denotes C₁-C₄-alkylen or C₂-C₄-alkenylen,
or a pharmaceutically acceptable salt thereof.

Also of interest are compounds of formula (I), wherein
- R¹: denotes ethyl, being substituted by a group selected from OH, OCH₃, -OCONHCH₂CH₃, -NHSO₂CH₃, -NHCOCH₃, morpholinyl and piperidinyl;
- R² and R³: together with the nitrogen form a 6 membered saturated or unsaturated heterocyclic ring which may contain nitrogen as an additional heteroatom, whilst the heterocyclic ring is substituted by a group selected from pyridinyl, phenyl and substituted phenyl being mono- or di-substituted by a group selected from CF₃, CH₃, OCH₃, F and Cl;
- A: denotes ethylen, propylen, butylen or butenylen,
or a pharmaceutically acceptable salt thereof.

Of particular interest are compounds of formula (I), wherein
- R¹: denotes ethyl, being substituted by a group selected from OH, OCH₃, - OCONHCH₂CH₃, -NHSO₂CH₃, -NHCOCH₃, morpholinyl and piperidinyl
- R² and R³: together with the nitrogen form a ring selected from the group consisting of piperazin, piperidin and tetrahydropyridin, which is substituted by a group selected from pyridinyl, phenyl and substituted phenyl being mono- or di-substituted by a group selected from CF₃, CH₃ and Cl;
- A: denotes ethylen, butylen or butenylen,
or a pharmaceutically acceptable salt thereof.

Furthermore preferred are compounds of formula (I), wherein
- R¹: denotes ethyl, being substituted by a group selected from OH, OCH₃, - OCONHCH₂CH₃ and -NHSO₂CH₃;
- R² and R³: together with the nitrogen form a piperazine-ring, being substituted by a group selected from trifluoromethylphenyl, methylphenyl, dimethylphenyl and chlorophenyl;
- A: denotes ethylen, butylen or butenylen,
or a pharmaceutically acceptable salt thereof.

The most preferred compounds disclosed in the invention are:
- 1-(2-methoxyethyl)-3-(4-{4-[3-(trifluoromethyl)-phenyl]-1-piperazinyl}-butyl)-1,3-dihydro-2H-benzimidazol-2-one;
- 1-{4-[4-(2,3-dimethylphenyl)-1-piperazinyl]-butyl}-3-(2-hydroxyethyl)-1,3-dihydro-2H-benzimidazol-2-one;
- 2-[2-oxo-3-(4-{4-[3-(trifluoromethyl)-phenyl]-1-piperazinyl}butyl)-2,3-dihydro-1H-benzimidazol-1-yl]-ethyl-ethylcarbamate;
- 1-(2-methoxyethyl)-3-(2-{4-[3-(trifluoromethyl)-phenyl]-1-piperazinyl}-ethyl)-1,3-dihydro-2H-benzimidazol-2-one;
- 1-{2-[4-(2,3-dimethylphenyl)-1-piperazinyl]-ethyl}-3-(2-methoxyethyl)-1,3-dihydro-2H-benzimidazol-2-one;
- 1-{2-[4-(3-chlorophenyl)-1-piperazinyl]-ethyl}-3-(2-hydroxyethyl)-1,3-dihydro-2H-benzimidazol-2-one;
- 2-[2-oxo-3-(2-{4-[3-(trifluoromethyl)-phenyl]-1-piperazinyl}-ethyl)-2,3-dihydro-1H-benzimidazol-1-yl]-ethyl-ethylcarbamate;
- 2-(3-{2-[4-(2,3-dimethylphenyl)-1-piperazinyl]-ethyl}-2-oxo-2,3-dihydro-1H-benzimidazol-1-yl)-ethyl-ethylcarbamate;
- N-[2-(3-{2-[4-(2,3-dimethylphenyl)-1-piperazinyl]-ethyl}-2-oxo-2,3-dihydro-1H-benzimidazol-1-yl)-ethyl]-methanesulfonamide;
- N-[2-(3-{(2Z)-4-[4-(3-methylphenyl)-1-piperazinyl]-2-butenyl}-2-oxo-2,3-dihydro-1H-benzimidazol-1-yl)-ethyl]-methanesulfonamide;
- N-[2-(3-{(2E)-4-[4-(3-chlorophenyl)-1-piperazinyl]-2-butenyl}-2-oxo-2,3-dihydro-1H-benzimidazol-1-yl)-ethyl]-methanesulfonamide;

If required, the compounds of general formulae (I) may be converted into the salts thereof, particularly, for pharmaceutical use, into the pharmaceutically acceptable salts thereof with an inorganic or organic acid. Suitable acids for this purpose include hydrochloric acid, hydrobromic acid, sulphuric acid, phosphoric acid, methanesulphonic acid, acetic acid, fumaric acid, succinic acid, lactic acid, citric acid, tartaric acid or maleic acid. Moreover, mixtures of these acids may be used.

The alkyl groups meant here (including those which are components of other groups) are branched and unbranched alkyl groups having 1 to 6 carbon atoms, preferably 1 to 4 carbon atoms, such as: methyl, ethyl, n-propyl, iso-propyl, butyl, iso-butyl sec.-butyl, tert.-butyl, pentyl, iso-pentyl and hexyl.

The alkylen groups meant here are branched and unbranched alkyl-bridges having 1 to 6 carbon atoms, preferably 1 to 4 carbon atoms, such as: methylen, ethylen, n-propylen, iso-propylen, butylen, iso-butylen, sec.-butylen, tert.-butylen, pentylen, iso-pentylen and hexylen.

Alkenyl groups (including those which are components of other groups) are the branched and unbranched alkenyl groups with 2 to 6 carbon atoms, preferably 2 to 4 carbon atoms, provided that they have at least one double bond, e.g. the alkyl groups mentioned above provided that they have at least one double bond, such as for example vinyl (provided that no unstable enamines or enolethers are formed), propenyl, iso-propenyl, butenyl, pentenyl and hexenyl.

Alkenylen groups are the branched and unbranched alkenyl-bridges with 2 to 6 carbon atoms, preferably 2 to 4 carbon atoms, provided that they have at least one double bond, e.g. the alkylen groups mentioned above provided that they have at least one double bond, such as for example vinylen (provided that no unstable enamines or enolethers are formed), propenylen, iso-propenylen, butenylen, pentenylen and hexenylen.

If not otherwise specified the alkenyl- and alkenylen-groups mentioned above are to be understood as embracing optionally existing stereoisomers. Accordingly, for instance the definition 2-butenyl is to be understood as embracing 2-(Z)-butenyl and 2-(E)-butenyl, etc..

The term alkynyl groups (including those which are components of other groups) refers to alkynyl groups having 2 to 6, preferably 2 to 4 carbon atoms provided that they have at least one triple bond, e.g. ethynyl, propargyl, butynyl, pentynyl and hexynyl.

Examples of N-linked 5- or 6-membered heterocyclic rings of general formula NR²R³ are as follows: pyrrole, pyrroline, pyrrolidine, piperidine, piperazine, morpholine, imidazole, imidazoline, imidazolidine, pyrazole, pyrazoline, pyrazolidine, preferably morpholine, piperazine and piperidine.

Examples of saturated or unsaturated bi- or tricyclic heterocyclic ring-system of formula NR²R³ which may contain nitrogen or oxygen as an additional heteroatom, are as follows: indole, tetrahydroindole, benzimidazole, benzoxazole, 1,2-dihydrochinoline, 1,2-dihydroisochinoline, β-carboline, 9H-1,2,3,4-tetrahydropyridoindole and 9,10-dihydroacridine.

Halogen, stands for fluorine, chlorine, bromine or iodine, preferably chlorine or bromine.

"=O" means an oxygen atom linked by a double bond.

The compounds of general formula (I) may be conveniently prepared by a variety of synthetic processes analogous to those known in the art using conventional methods. For example these compounds may be prepared by alkylating the suitable secondary amine (III) with the proper benzimidazolone (II) bearing in the alkyl or alkenyl side chain suitable leaving group X such as halogen, methansulfonate or 4-methylbenzenesulfonate (scheme 1).

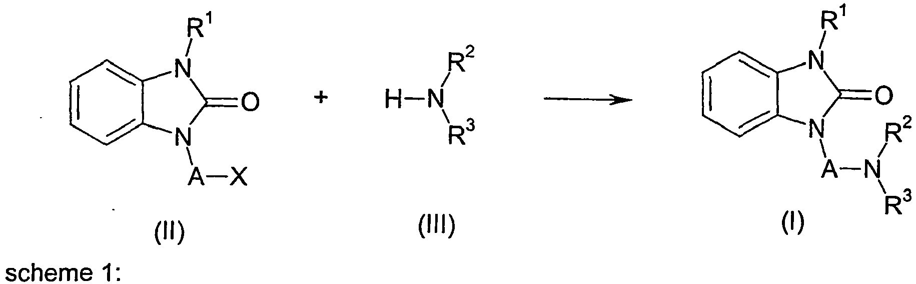

The reaction conditions for the conventional synthesis of compounds of formula (I) according to scheme 1 are disclosed in EP 526 434 A1. Said reference additionally describes the possible synthetic pathways for the preparation of starting compounds (II).

According to a second option, the reaction sequence according to scheme 1 can not only be conducted via the conventional synthetic methods outlined in EP 526 434 A1 but, in the alternative, via combinatorial chemistry. For this approach a set of N-alkyl-N' halo alkyl/alkenyl benzimidazolones of formula (II) (from now on identified as Building Blocks or BB; see hereto table 1) was prepared via the traditional methods descirbed in EP 526 434 A1 and then combinatorially reacted with the suitable secondary amines of formula (III) (table 2).

The process was carried out in a special apparatus consisting of a lower vial (reacting chamber) and an upper vial (condenser). Each compound was reacted with each amine in DMF under stirring at a temperature between 40 and 100°C preferably at 60 °C for 6 to 8 hours in the presence of Na₂CO₃. The excess amine was then scavenged at room temperature by introducing a polystyrene isocyanatemethyl resin of formula (IV) able to catch the excess amine as an urea of formula (V) immobilised on the solid support (scheme 2).

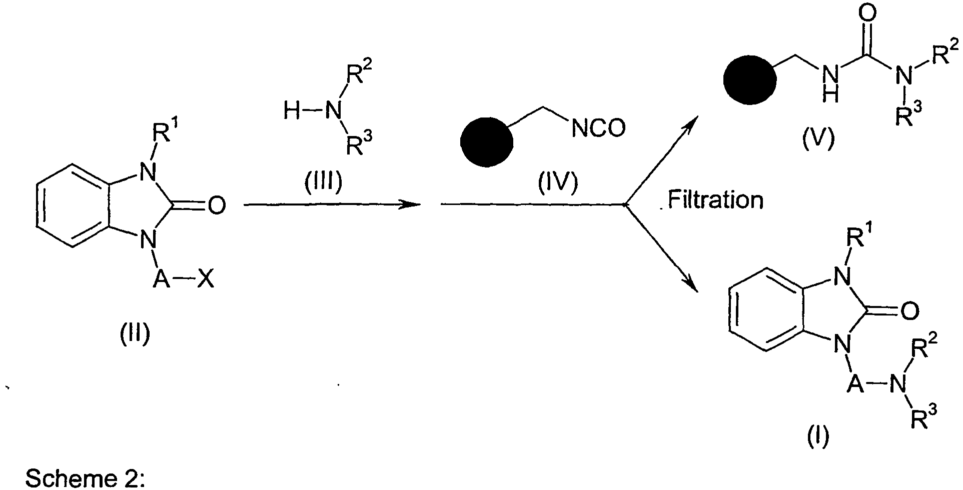

The upper part of the reaction apparatus is substituted with another vial containing a frit inside and a connection to the vacuum. Filtration after turning over the apparatus and evaporation to dryness afforded the desired compounds of formula (I) in excellent yield and good purity. The parallel application of the aforementioned process to all of the compounds of formula (II) as shown in table 1 and all of the selected amines (III) as shown in table 2 allows the efficient synthesis of all of the compounds (I) according to the present invention.

**Table 1: building blocks (BB) of formula (II) subjected to the process according to scheme 2**

| | | | |
|---|---|---|---|
| 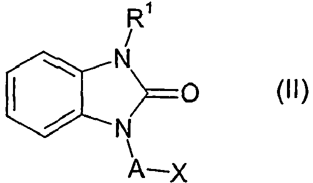 | | | |

| Building Block No. | Structure | Building Block No. | Structure |
|---|---|---|---|
| BB01 | 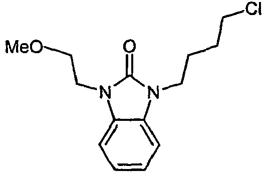 | BB02 | 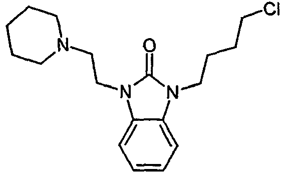 |
| BB03 | 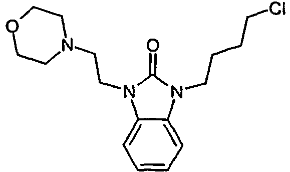 | BB04 | 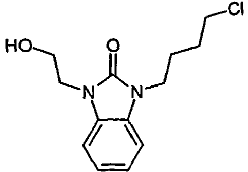 |
| BB05 | 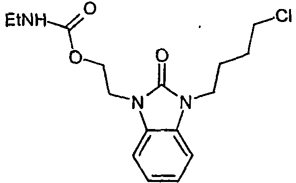 | BB06 | 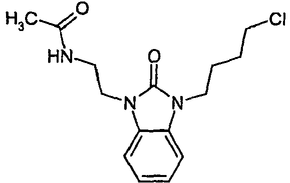 |
| BB07 | 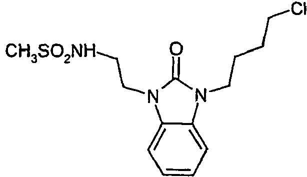 | BB08 | 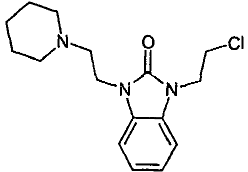 |
| BB09 | 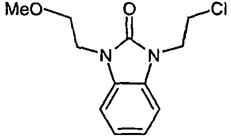 | BB10 | 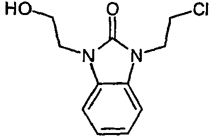 |
| BB11 | 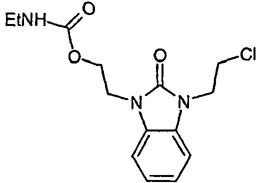 | BB12 | 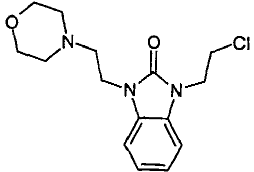 |
| BB13 | 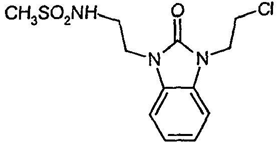 | BB14 | 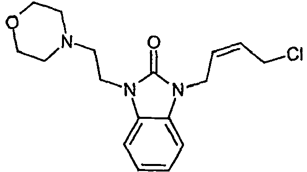 |
| BB15 | 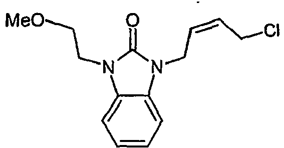 | BB16 | 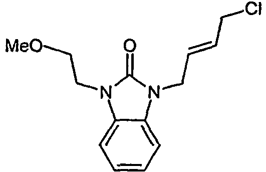 |
| BB17 | 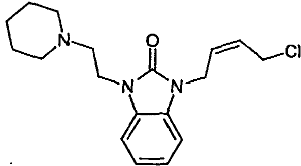 | BB18 | 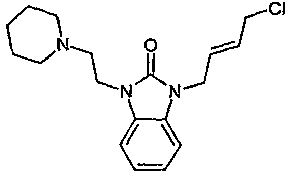 |
| BB19 | 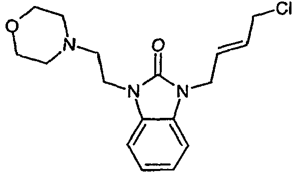 | BB20 | 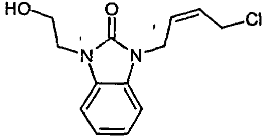 |
| BB21 | 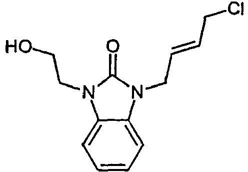 | BB22 | 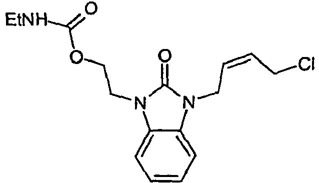 |
| BB23 | 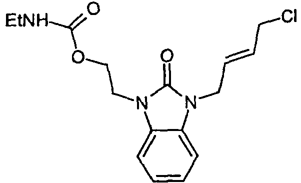 | BB24 | 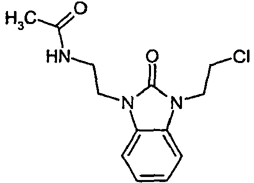 |
| BB25 | 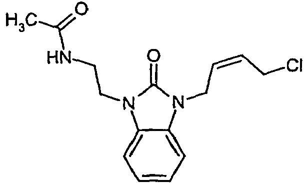 | BB26 | 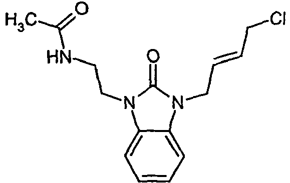 |
| BB27 | 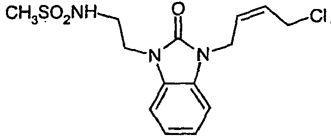 | BB28 | 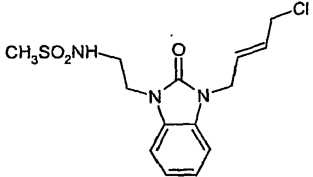 |

**Table 2: Amines (AM) of formula (III) subjected to the process according to scheme 2**

| | | | |
|---|---|---|---|
| 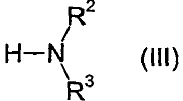 | | | |

| Amine No. | Structure | Amine No. | Structure |
|---|---|---|---|
| AM01 | 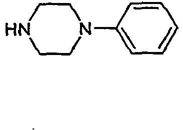 | AM02 | 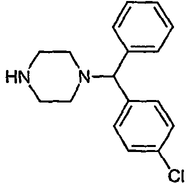 |
| AM03 | 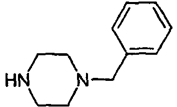 | AM04 | 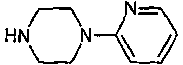 |
| AM05 | 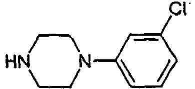 | AM06 | 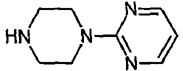 |
| AM07 | 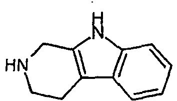 | AM08 | 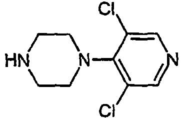 |
| AM09 | 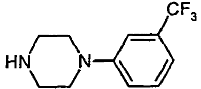 | AM10 | 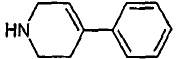 |
| AM11 | 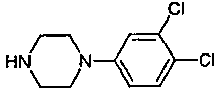 | AM12 | 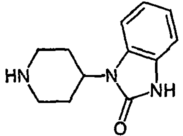 |
| AM13 | 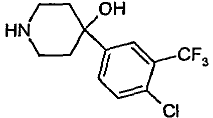 | AM14 | 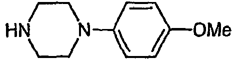 |
| AM15 | 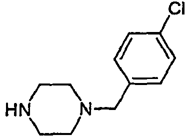 | AM16 | 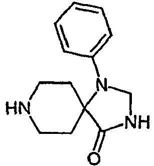 |
| AM17 | 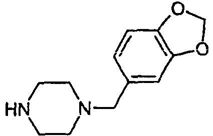 | AM18 | 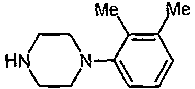 |
| AM19 | 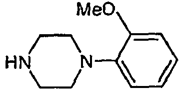 | AM20 | 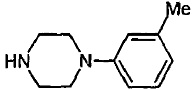 |
| AM21 | 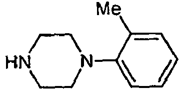 | AM22 | 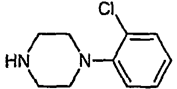 |

For the pharmaceutical use the compounds of general formula (I) may be used as such or in the form of physiologically acceptable acid addition salts. The term "acid acceptable salts" includes both organic and inorganic acids such as maleic, citric tartaric, methanesulphonic, acetic, benzoic, succinic, gluconic, isethionic, glycinic, lactic, malic, mucoic, glutammic, sulphamic and ascorbic acids; inorganic acids include hydrochloric, hydrobromic, nitric, sulfuric, or phosphoric acid.

According to a further feature of the present invention there are provided pharmaceutical compositions comprising as an active ingredient at least one compound of formula (I), as before defined, or a physiologically acceptable addition salt thereof in additon with one or more pharmaceutical carrier, diluents or excipients. For pharmaceutical administration the compounds of general formula (I) and their physiologically acceptable acid addition salts may be incorporated into the conventional pharmaceutical preparation in solid, liquid or spray form. The composition may, for example, be presented in a form suitable for oral, rectal, parenteral administration or for nasal inhalation: preferred forms includes for example, capsules, tablets, coated tables, ampoules, suppositories and nasal spray. The active ingredient may be incorporated in excipients or carriers conventionally used in pharmaceutical compositions such as, for example, talc, arabic gum, lactose, gelatine, magnesium stearate, corn starch, aqueous or non aqueous vehicles, polyvynil pyrrolidone, semisynthetic glicerides of fatty acids, benzalcon chloride, sodium phosphate , EDTA, polysorbate 80.

In case it is desired to further increase the solubilty of the compounds of general formula (I) or of their physiologically acceptable salts surfactants, non ionic surfactants such as PEG 400, cyclodextrin, metastable polymorphs, inert adsorbents such as bentonite, may be incorporated. Furthermore some techniques may be employed by preparing for example eutectic mixtures and/or solid dispersion by using mannitol, sorbitol, saccharose, succinic acid or physical modified forms by using hydrosoluble polymers, PVP, PEG 4000-20.000.

The compositions are advantageously formulated in dosage units, each dosage unit being adapted to supply a single dose of the active ingredient. Each dosage unit may conveniently contain from 0,01 mg to 100 mg, preferably from 0,1 to 50 mg.

However, it could be necessary to depart from the cited amounts, depending on the body weight or on the administration route, on the individual response to the medicament, on the type of formulation and on the time, or time range, in which the administration is carried out. Therefore, it can be sufficient, in some cases, to use a lower amount then the cited minimum amount, whereas in other cases the higher range could be exceeded. When administering higher amounts, it would be advisable to subdivide them in repeated administrations during the day. Moreover, the compounds of general formula (I) or the acid addition salts thereof can also be combined with other, different active substances.

The following examples illustrate the present invention, without limiting the scope thereof.

### Examples of pharmaceutical formulation

| A) | Tablets | for tablet |
|---|---|---|
| | Active substance | 100 mg |
| | Lactose | 140 mg |
| | Maize starch | 240 mg |
| | Polyvinylpyrrolidone | 15 mg |
| | Magnesium stearate | 5 mg |
| | | 500 mg |

The finely ground active substance, lactose are part of maize starch are mixed. The mixture is sieved, wetted with a solution of polyvinylpyrrolidone in water, kneaded, finely granulated and dried. The granulate, the remaining maize starch and magnesium stearate are sieved and mixed together. The mixture is compressed to tablets of suitable form and size.

| B) | Tablets | for tablet |
|---|---|---|
| | Active substance | 80 mg |
| | Lactose | 55 mg |
| | Maize starch | 190 mg |
| | Microcrystalline cellulose | 35 mg |
| | Polyvinylpyrrolidone | 15 mg |
| | Sodium carboxymethyl starch | 23 mg |
| | Magnesium stearate | 2 mg |
| | | 400 mg |

The finely ground active substance, part of the maize starch, lactose, microcrystalline cellulose and polyvinylpyrrolidone are mixed. The mixture is sieved and worked up with the remaining maize starch and water, to obtain a granulate, which is dried and sieved. This is added with sodium carboxymethyl starch and magnesium stearate and mixed, then the mixture is compressed to tablets of suitable size.

| C) | Solution for vials | |
|---|---|---|
| | Active substance | 50 mg |
| | Sodium chloride | 50 mg |
| | Water for injection | 5 ml |

The active substance is dissolved in water, optionally at pH of 5.5 to 6.5, and treated with sodium chloride as an osmolality agent. The resulting solution is filtered apyrogenically, and the filtrate is placed in vials under asepsis conditions, then vials are sterilized and flame sealed. The vials contain 5 mg, 25 mg and 50 mg of active substance.

### Experimental part

The following examples illustrate the preparation of all the new compounds included in the present invention. It should be understood that the invention is not limited to the given examples of chemical methods and processes for the preparation of the substances, as other conventional methods well known to those skilled in the art, are suitable too. In the following descriptions, each of the 28 Building Blocks prepared is identified by its relevant Tag.

### Part A) Preparation of the Building Blocks (BB) of formula (II)

### Description 1

### 1-[2-(1-piperidinyl)-ethyl)-1,3-dihydro-2H-benzimidazol-2-one

A solution of 1-isopropenyl-1,3-dihydro-2H-benzimidazol-2-one (35 g, 0.2 moles) in DMF (250 ml) was added dropwise to a suspension of 80% sodium hydride (6 g, 0.2 moles) in DMF (50 ml). The reaction mixture was first heated at 45 °C for 30 minutes, let to cool at room temperature and additional 80 % sodium hydride (7.2 g, 0.24 moles) was added. Then 1-(2-chloroethyl)-piperidine hydrochloride (44.16 g, 0.24 moles) was added portionwise and the reaction mixture was heated at 80-90 °C for 3 hrs. The mixture was then cooled at room temperature, adjusted to pH 3 with 37% aqueous HCl and heated at 80 °C for additional 2 hrs. The reaction mixture was poured into water and washed with ethyl acetate. The aqueous phase was adjusted to pH 8-9 with a saturated sodium carbonate solution and extracted into ethyl acetate. The organic layer was taken to dryness to give an ivory solid which after crystallization from isopropyl ether afforded 22.9 g of the title compound. m.p. 123-125 °C.

According to the above described procedure, the following compound was prepared from the suitable intermediates:

### 1-[2-(4-morpholinyl)-ethyl]-1,3-dihydro-2H-benzimidazol-2-one

11.7 g; m.p. 122-126 °C.

### Description 2

### 1-(2-methoxyethyl)-1,3-dihydro-2H-benzimidazol-2-one

Phenyl-2-oxo-2,3-dihydro-1H-benzimidazole-1-carboxylate (80 g, 0.315 moles) was added to a suspension of 80% sodium hydride (11.3 g, 0.378 moles) in DMF (500 ml) and heated at 35 °C for 1 hour. To the cooled solution 2-chloroethyl-methylether (43 ml, 0.472 moles) was added and the reaction mixture was heated at 100 °C for 4 hrs. The reaction mixture was poured into water and extracted with ethyl acetate. The combined extracts were taken to dryness to give 52 g of the protected intermediate. This was suspended in methanol (500 ml), a solution of K₂CO₃ (44 g) in water (230 ml) was added and the mixture and stirred for 2 hrs at room temperature. After evaporation the reaction mixture was acidified and extracted into ethyl acetate. The organic layer was taken to dryness and from the crude oily residue, after crystallization with isopropyl ether 21 g of the title compound were obtained as a white solid. m.p. 88 °C.

### Description 3

### 1-[2-(tetrahydro-2H-pyran-2-yloxy)-ethyl]-1,3-dihydro-2H-benzimidazol-2-one

a) Phenyl-2-oxo-2,3-dihydro-1H-benzimidazole-1-carboxylate (50 g, 0.197 moles) was added to a suspension of 80% sodium hydride (7 g, 0.236 moles) in DMF (400 ml) and stirred for 1 hour at room temperature, then 2-(2 chloro-ethoxy)-tetrahydro-2H-pyran (34.8 ml, 0.236 moles) was added and the reaction mixture was heated at 100 °C for 7 hrs. The reaction mixture was then poured into water and extracted into ethyl acetate. The organic layer was taken to dryness to give an oily residue.
b) This residue (83 g) was dissolved in methanol, a solution of KOH (26 g in 260 ml water) was added and stirred for 2 hrs at room temperature. The methanol was evaporated and the residue was extracted into ethyl acetate. The organic layer was washed with an aqueous 5% HCl solution, dried and taken to dryness. The oily residue was crystallized from diisopropyl ether to give 26 g of the title compound. m.p. 115 °C.

### Description 4

### 1-(2-aminoethyl)-1,3-dihydro-2H-benzimidazol-2-one hydrochloride

a) A suspension of Phenyl-2-oxo-2,3-dihydro-1H-benzimidazole-1-carboxylate (10 g, 39 mmoles) and 80% sodium hydride (1.3 g, 43 mmoles) in DMF (100 ml) was stirred at room temperature for 30 minutes. N-(2-bromoethyl)-phtalimide (10 g, 39 mmoles) was added and the mixture heated at 100 °C for 12 hrs. The reaction mixture was then poured into 600 ml of water and stirred for 4 hrs at room temperature. The precipitated phthaloyl derivative was filtered off (white solid; 6.5 g).
b) This intermediate was suspended in methanol (70 ml), a 10% aqueous K₂CO₃ solution was added and the reaction mixture stirred overnight at room temperature. The methanol was evaporated, the residue was extracted with dichloro methane and the aqueous solution was acidified with 10% aqueous HCl to give the corresponding 2-carboxy-benzamido derivative which was filtered off (5g).
c) To the crude intermediate were added 32 ml of a 15% aqueous HCl solution and the resulting suspension was heated at 90 °C for 3 hrs. After cooling, the solid was filtered off and the acidic solution was taken to dryness to give 1.5 g of the hydrochloride of the title compound as a pinkish solid. m.p. > 280 °C.

### Description 5

### N-[2-(2-oxo-2,3-dihydro-1H-benzimidazol-1-yl)-ethyl]-acetamide

To a cooled solution of NaOH (0.41 g, 10 mmoles) in water (5 ml) were simultaneously added 1-(2-aminoethyl)-1,3-dihydro-2H-benzimidazol-2-one hydrochloride (0.7 g, 3.3 mmoles) and a solution of acetic anhydride (0.37 ml, 3.9 mmoles) in dioxane (10 ml). The reaction mixture was stirred for 2 hrs at room temperature and then taken to dryness. The residue was dissolved in water, adjusted to pH 4 with 10% aqueous HCl and extracted with CHCl₃. The organic layer was taken to dryness and from the crude residue 0.35 g of the title compound were obtained after crystallization from diethyl ether. m.p. 153 °C.

### Description 6

### N-[2-(2-oxo-2,3-dihydro-1H-benzimidazol-1-yl)-ethyl]-methanesulfonamide

To a solution of 1-(2-aminoethyl)-1,3-dihydro-2H-benzimidazol-2-one hydrochloride (2.8 g, 13 mmoles) in THF (30 ml) and triethyl amine (5.5 ml, 39 mmoles) was added methanesulfonyl chloride (1.12 ml, 14 mmoles) and the reaction mixture was stirred for 2 hrs at room temperature. The organic solvent was evaporated and the residue was partitioned into water and ethyl acetate. The organic layer was washed with saturated aqueous Na₂CO₃ solution and taken to dryness. The crude residue was purified by flash chromatography (CH₂Cl₂-methanol 96-4) to give 0.5 g of the title compound as a white solid. m.p. 162-170 °C.

### Description 7

### 1-(2-chloroethyl)-3-[2-(tetrahydro-2H-pyran-2-yloxy)-ethyl]-1,3-dihydro-2H-benzimidazol-2-one

Into a stirred solution of 1-[2-(tetrahydro-2H-pyran-2yloxy)-ethyl]-1,3-dihydro-2H-benzimidazol-2-one (6.5 g, 25 mmoles) in DMF (40 ml) 80% sodium hydride (0.9 g, 30 mmoles) was added. After 30 minutes of stirring and heating 35 °C, 1-bromo-2-chloro ethane (6.2 g, 48 mmoles) was added, the reaction temperature was increased at 90 °C and kept for 6 hrs then cooled at room temperature. The reaction mixture was poured into water, extracted with diethyl ether and the organic layer was taken to dryness. The residue was purified by flash chromatography (cyclohexane-ethyl acetate 50-50) to give 2.8 g of the title compound as a thick oil which was used without any further purification.

According to the above described procedure, the following compounds were prepared from the suitable intermediates:

### 1-(4-chlorobutyl)-3-[2-(tetrahydro-2H-pyran-2-yloxy)-ethyl]-1,3-dihydro-2H-benzimidazol-2-one

The compound was purified by flash chromatography (cyclohexane-ethyl acetate 50-50). Thick oil.

### [BB09]: 1-(2-chloroethyl)-3-(2-methoxyethyl)-1,3-dihydro-2H-benzimidazol-2-one

White solid. m.p. 55 °C., from diisopropyl ether

### [BB01]: 1-(4-chlorobutyl)-3-(2-methoxyethyl)-1,3-dihydro-2H-benzimidazol-2-one

The compound was purified by flash chromatography (cyclohexane-ethyl acetate 50-50). Thick oil.

### [BB15]: 1-[(2Z)-4-chloro-2butenyl)]-3-(2-methoxyethyl)-1,3-dihydro-2H-benzimidazol-2-one

The compound was purified by flash chromatography (cyclohexane-ethyl acetate 70-30). Thick oil.

### [BB24]: N-{2-[3-(2-chloroethyl)-2-oxo-2,3-dihydro-1H-benzimidazol-1-yl]-ethyl}-acetamide

White solid. m.p. 140-142 °C., from acetone

### [BB06]: N-{2-[3-(4-chlorobutyl)-2-oxo-2,3-dihydro-1H-benzimidazol-1-yl]-ethyl}-acetamide

Ivory solid. m.p. 100 °C., from diethyl ether

### Description 8

### 1-[(2Z)-4-chloro-2-butenyl]-3-[2-(tetrahydro-2H-pyran-2-yloxy)-ethyl]-1,3-dihydro-2H-benzimidazol-2-one

To a solution of 1-[2-(tetrahydro-2H-pyran-2yloxy)-ethyl]-1,3-dihydro-2H-benzimidazol-2-one (12 g, 46 mmoles) in DMF (120 ml) was added 80% sodium hydride (1.7 g, 55 mmoles) and the mixture was stirred at room temperatute for 1 hour. Cis 1,4-dichloro-2-butene (5.8 ml, 55 mmoles) was dropped in and the reaction mixture was stirred for 3 hrs at room temperature. The reaction mixture was poured into water and extracted with ethyl acetate. The organic layer was taken to dryness and the residue was purified by flash chromatography (cyclohexane-ethyl acetate 70-30) to give 2.8 g of the title compound as a thick oil which was used without any further purification.

According to the above described procedure, the following compounds were prepared from the suitable intermediates:

### 1-[(2E)-4-chloro-2-butenyl)]-3-[2-(tetrahydro-2H-pyran-2-yloxy)-ethyl]-1,3-dihydro-2H-benzimidazol-2-one

The compound was purified by flash chromatography (cyclohexane-ethyl acetate 70-30). Waxy solid.

### [BB1 6]: 1-[(2E)-4-chloro-2-butenyl)]-3-(2-methoxyethyl)-1,3-dihydro-2H-benzimidazol-2-one

The compound was purified by flash chromatography (cyclohexane-ethyl acetate 70-30). Thick oil.

### [BB25]: N-(2-{3-[(2Z)-4-chloro-2-butenyl]-2-oxo-2,3-dihydro-1H-benzimidazol-1-yl]}-ethyl)-acetamide

The compound was purified by flash chromatography (CH₂Cl₂-methanol 97-3). Waxy solid from diisopropyl ether, m.p. 118 °C.

### [BB26]: N-(2-{3-[(2E)-4-chloro-2-butenyl]-2-oxo-2,3-dihydro-1H-benzimidazol-1-yl]}-ethyl)-acetamide

White solid from diethyl ether, m.p. 108 °C.

### [BB13]: N-{2-[3-(2-chloroethyl)-2-oxo-2,3-dihydro-1H-benzimidazol-1-yl]-ethyl}-methanesulfonamide

The compound was purified by flash chromatography (CH₂Cl₂-methanol 98-2). White low melting solid.

### [BB07]: N-{2-[3-(4-chlorobutyl)-2-oxo-2,3-dihydro-1H-benzimidazol-1-yl]-ethyl}-methanesulfonamide

White solid, m.p. 104 °C from diethyl ether.

### [BB27]: N-(2-{3-[(2Z)-4-chloro-2-butenyl)]-2-oxo-2,3-dihydro-1H-benzimidazol-1-yl]-ethyl}-methanesulfonamide

The compound was purified by flash chromatography (CH₂Cl₂-methanol 97-3). White solid, m.p. 83 °C from diethyl ether.

### [BB28]: N-2-{3-[(2E)-4-chloro-2butenyl)]-2-oxo-2,3-dihydro-1H-benzimidazol-1-yl]-ethyl}-methanesulfonamide

The compound was purified by flash chromatography (CH₂Cl₂-methanol 98-2). White solid, m.p. 98 °C from diethyl ether.

### Description 9

### [BB08]: 1-(2-chloroethyl)-3-[(2-piperidinyl)-ethyl]-1,3-dihydro-2H-benzimidazol-2-one

A solution of 1-[2-(1-piperidinyl)-ethyl)-1,3-dihydro-2H-benzimidazol-2-one (4 g, 16.3 mmoles) in DMF (50 ml) was added to a suspension of 80% sodium hydride (0.49 g, 16.3 mmoles) in DMF (25 ml) and the reaction mixture was heated under stirring for 30 minutes at 40 °C. The solution was slowly transferred (3 hrs) into a solution of 1-bromo-2-chloro-ethane (2.7 ml, 32.6 mmoles) in DMF (30 ml), the temperature was increased to 60 °C and stirred for 5 hrs. The reaction mixture was then taken to dryness under vacuum, the residue partitioned between 5% aqueous HCl and diethyl ether. The aqueous layer was adjusted to pH 9÷10 with sodium carbonate and extracted with ethy acetate. After evaporation and flash chromatography purification (CH₂Cl₂-methanol-NH₄OH 95-5-0.5) 2.2 g of the pure title compound was obtained as a clear oil.

According to the above described procedure, the following compounds were prepared from the suitable intermediates:

### [BB02]: 1-(4-chlorobutyl)-3-[(2-(1-piperidinyl)-ethyl]-1,3-dihydro-2H-benzimidazol-2-one

The compound was purified by flash chromatography (CH₂Cl₂-methanol-NH₄OH 95-5-0.5). Ivory solid, m.p. 82-87 °C from diethyl ether.

### [BB12]: 1-(2-chloroethyl)-3-[2-(4-morpholinyl)-ethyl]-1,3-dihydro-2H-benzimidazol-2-one

The compound was purified by flash chromatography (CH₂Cl₂-methanol-NH₄OH 95-5-0.5). Thick oil.

### [BB03]: 1-(4-chlorobutyl)-3-[2-(4-morpholinyl)-ethyl]-1,3-dihydro-2H-benzimidazol-2-one

The compound was purified by flash chromatography (CH₂Cl₂-methanol-NH₄OH 95-5-0.5). Clear oil.

### Description 10

### [BB14]: 1-[(2Z)-4-chloro-2-butenyl)]-3-[2-(4-morpholinyl)-ethyl]-1,3-dihydro-2H-benzimidazol-2-one

A solution of 1-[2-(4-morpholinyl)-ethyl]-1,3-dihydro-2H-benzimidazol-2-one (4 g, 16.2 mmoles) in DMF (50 ml) was added dropwise to a suspension of 80% sodium hydride (0.49 g, 16.2 mmoles) in DMF (50 ml) and the mixture was heated under stirring at 45 °C for 30 minutes. This solution was slowly transferred (4 hrs) to a solution of cis-1,4-dichloro-2-butene (3.43 ml, 32.4 mmoles) in DMF (20 ml). The reaction mixture was stirred overnight at room temperature, taken to dryness under vacuum and partitioned between ethyl acetate and water. From the organic solution after evaporation and flash chromatography purification (CH₂Cl₂-methanol-NH₄OH 95-5-0.5) 2.4 g of the title compound were obtained as an oil.

According to the above described procedure, the following compounds were prepared from the suitable intermediates:

### [BB19]: 1-[(2E)-4-chloro-2-butenyl]-3-[2-(4-morpholinyl)-ethyl]-1,3-dihydro-2H-benzimidazol-2-one

The compound was purified by flash chromatography (CH₂Cl₂-methanol-NH₄OH 95-5-0.5). Thick oil.

### [BB17]: 1-[(2Z)-4-chloro-2-butenyl]-3-[2-(1-piperidinyl)-ethyl]-1,3-dihydro-2H-benzimidazol-2-one

The compound was purified by flash chromatography (CH₂Cl₂-methanol-NH₄OH 95-5-0.5). Thick oil.

### [BB18]: 1-[(2E)-4-chloro-2-butenyl]-3-[2-(1-piperidinyl) -ethyl]-1,3-dihydro-2H-benzimidazol-2-one

The compound was purified by flash chromatography (CH₂Cl₂-methanol-NH₄OH 95-5-0.5). Thick oil.

### Description 11

### [BB10]: 1-(2-chloroethyl)-3-(2-hydroxyethyl)-1,3-dihydro-2H-benzimidazol-2-one

A solution of 1-(2-chloroethyl)-3-[2-(tetrahydro-2H-pyran-2-yloxy)-ethyl]-1,3-dihydro-2H-benzimidazol-2-one (2.2 g) and a catalytic amount of p-toluensulfonic acid (0.1 g) in methanol (30 ml) was stirred at room temperature for 2 hrs. The reaction mixture was evaporated to dryness, the residue was dissolved in CH₂Cl₂ and washed with a saturated aqueous solution of K₂CO₃. The organic layer was taken to dryness to give 1.5 g of the title compound as white solid. m. p. 135 °C.

According to the above described procedure, the following compounds were prepared from the suitable intermediates:

### [BB04]: 1-(4-chlorobutyl)-3-(2-hydroxyethyl)-1,3-dihydro-2H-benzimidazol-2-one

Thick oil.

### [BB20]: 1-[(2Z)-4-chloro-2-butenyl]-3-(2-hydroxyethyl)-1,3-dihydro-2H-benzimidazol-2-one

White solid, m. p. 80 °C from diethyl ether.

### [BB21]: 1-[(2E)-4-chloro-2-butenyl]-3-(2-hydroxyethyl)-1,3-dihydro-2H-benzimidazol-2-one

Ivory solid, m. p. 73 °C from diethyl ether.

### Description 12

### [BB11]: 2-[3-(2-chloroethyl)-2-oxo-2,3-dihydro-1H-benzimidazol-1-yl]-ethyl-ethylcarbamate

1-(2-chloroethyl)-3-(2-hydroxyethyl)-1,3-dihydro-2H-benzimidazol-2-one (2.6 g, 11 mmoles) and ethyl isocyanate (20 ml) were refluxed under stirring for 6 hrs then left overnight at room temperature. The reaction mixture was taken to dryness and the residue was crystallized from diisopropyl ether to give 3 g of the title compound. m. p. 125 °C.

According to the above described procedure, the following compound was prepared from the suitable intermediate:

### [BB05]: 2-[3-(4-chlorobutyl)-2-oxo-2,3-dihydro-1H-benzimidazol-1yl]-ethyl-ethylcarbamate

White solid, m. p. 75 °C from diethyl ether.

### Description 13

### [BB22]: 2-{3-[(2Z)-4-chloro-2-butenyl]-2-oxo-2,3-dihydro-1H-benzimidazol-1-yl}-ethyl-ethylcarbamate

1-(4-chlorobutyl)-3-(2-hydroxyethyl)-1,3-dihydro-2H-benzimidazol-2-one (1.8 g, 6.8 mmoles) and ethyl isocyanate (6 ml) were stirred at room temperature for 48 hrs. The reaction mixture was then taken to dryness and the residue was crystallized from diethyl ether to give 1.8 g the title compund. m. p. 107 °C.

According to the above described procedure, the following compound was prepared from the suitable intermediate:

### [BB23]: 2-{3-[(2E)-4-chloro-2-butenyl]-2-oxo-2,3-dihydro-1H-benzimidazol-1-yl}-ethyl-ethylcarbamate

The compound was purified by flash chromatography (cyclohexane-ethyl acetate 50-50). White solid, m. p. 70 °C from diethyl ether.

### Part B) General method for the preparation of the compounds of formula (I)

A solution of each building block (II) (0,1mM) was reacted under stirring with each amine (0,2 mM) in anhydrous DMF (100 µl) in the presence of Na₂CO₃ (0,3 mM) at a temperature ranging from room temperature to 100 °C, preferably between 60 °C and 80 °C, for about 6-8 hours. Isocyanatemethyl Polystyrene Resin (loading 0,23 meq/g), (0,2 mM) was introduced and the mixture was gently stirred at room temperature for 8 hours. The resin was then filtered off under vacuum, washed with DMF, and filtered again. The collected solutions were evaporated to dryness in a speed-vac centrifuge. The compounds which were prepared according to the above described procedure are listed in Table 3.

Table 3 collects the structural formula of the synthesised compounds along with the corresponding characterising mass data (i.e. [M+H]⁺) obtained for each of the compounds according to the invention. The identification of the compounds and their purity was carried out by using positive APCI-LC/MS technique.

**Table 3: compounds of general formula (I),**

| | | | | | |
|---|---|---|---|---|---|
| 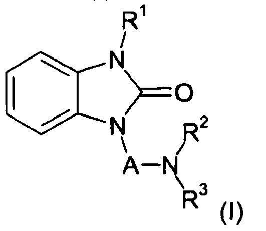 | | | | | |

| Comp. No | -R¹ | -A- | 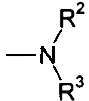 | [M+H]⁺ | R*/I* |
|---|---|---|---|---|---|
| 1 | 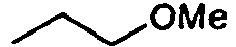 | 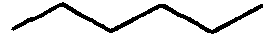 | 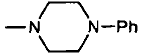 | 409 | I |
| 2 | 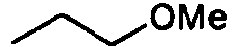 | 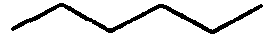 | 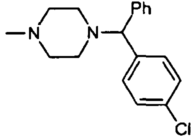 | 533 | I |
| 3 | 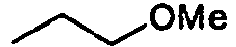 | 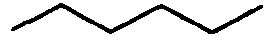 | 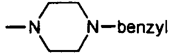 | 423 | I |
| 4 | 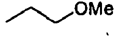 | 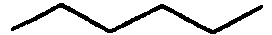 | 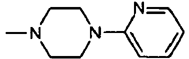 | 410 | I |
| 5 | 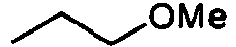 | 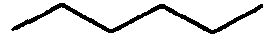 | 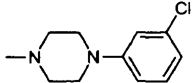 | 443 | I |
| 6 | 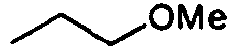 | 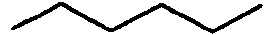 | 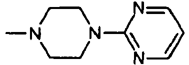 | 411 | I |
| 7 | 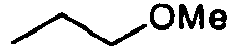 | 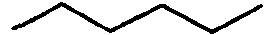 | 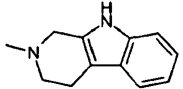 | 419 | I |
| 8 | 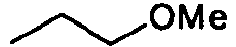 | 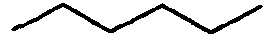 | 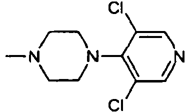 | 478 | I |
| 9 | 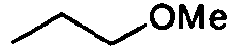 | 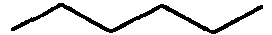 | 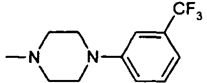 | 477 | I |
| 10 | 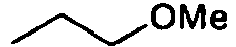 | 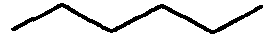 | 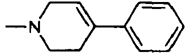 | 406 | I |
| 11 | 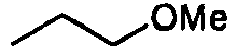 | 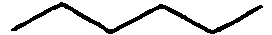 | 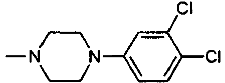 | 477 | I |
| 12 | 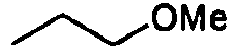 | 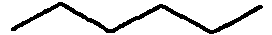 | 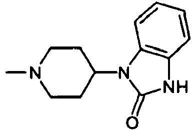 | 464 | I |
| 13 | 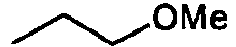 | 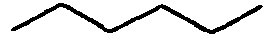 | 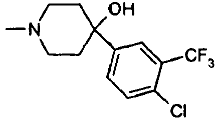 | 526 | I |
| 14 | 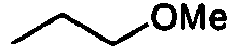 | 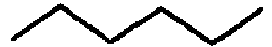 | 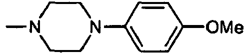 | 439 | I |
| 15 | 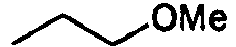 | 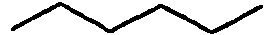 | 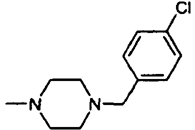 | 457 | I |
| 16 | 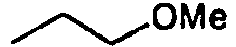 | 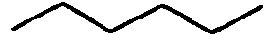 | 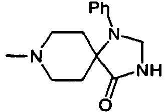 | 478 | I |
| 17 | 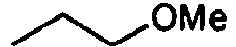 | 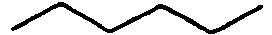 | 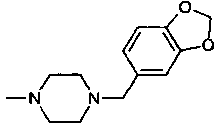 | 467 | I |
| 18 | 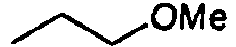 | 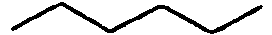 | 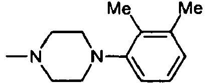 | 437 | I |
| 19 | 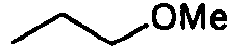 | 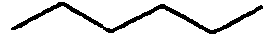 | 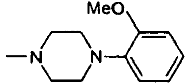 | 439 | I |
| 20 | 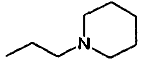 | 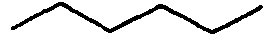 | 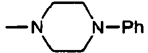 | 462 | R |
| 21 | 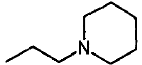 | 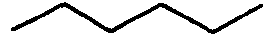 | 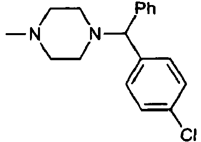 | 586 | R |
| 22 | 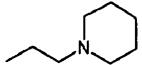 | 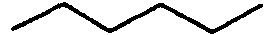 | 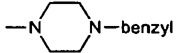 | 476 | R |
| 23 | 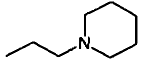 | 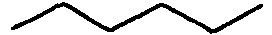 | 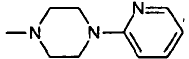 | 463 | R |
| 24 | 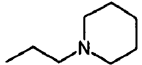 | 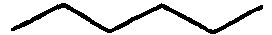 | 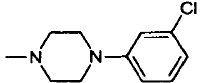 | 496 | R |
| 25 | 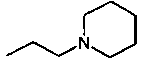 | 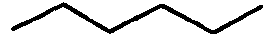 | 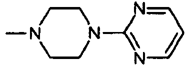 | 464 | R |
| 26 | 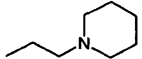 | 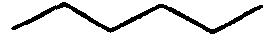 | 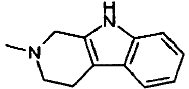 | 472 | R |
| 27 | 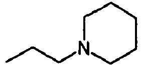 | 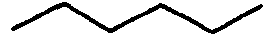 | 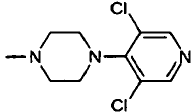 | 531 | R |
| 28 | 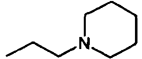 | 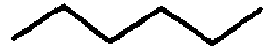 | 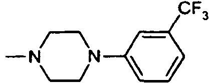 | 530 | R |
| 29 | 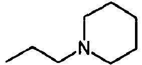 | 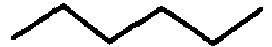 | 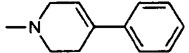 | 459 | R |
| 30 | 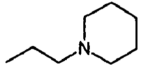 | 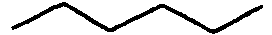 | 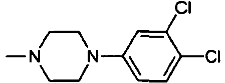 | 530 | R |
| 31 | 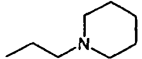 | 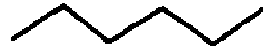 | 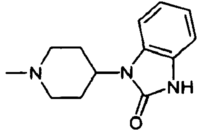 | 517 | R |
| 32 | 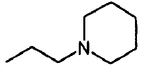 | 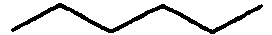 | 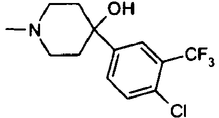 | 579 | R |
| 33 | 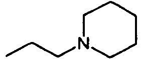 | 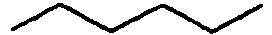 | 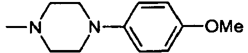 | 492 | R |
| 34 | 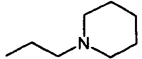 | 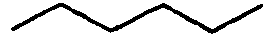 | 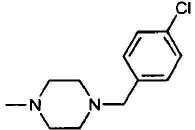 | 510 | R |
| 35 | 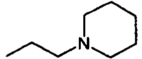 | 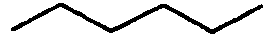 | 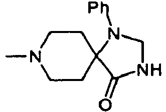 | 531 | R |
| 36 | 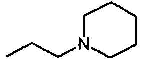 | 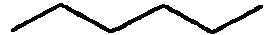 | 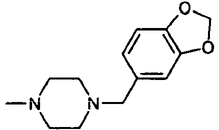 | 520 | R |
| 37 | 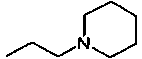 | 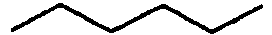 | 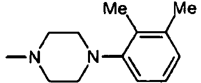 | 490 | R |
| 38 | 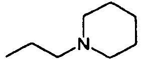 | 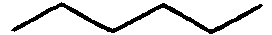 | 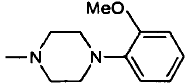 | 492 | R |
| 39 | 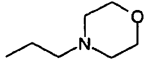 | 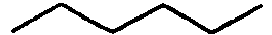 | 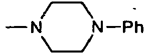 | 464 | R |
| 40 | 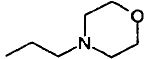 | 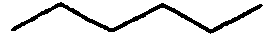 | 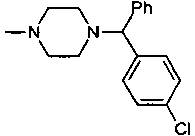 | 588 | R |
| 41 | 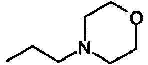 | 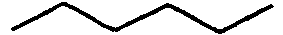 | 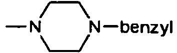 | 478 | R |
| 42 | 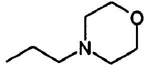 | 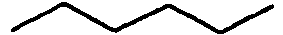 | 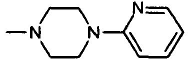 | 465 | R |
| 43 | 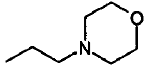 | 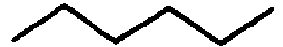 | 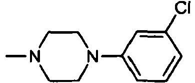 | 498 | R |
| 44 | 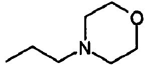 | 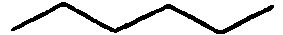 | 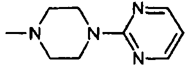 | 466 | R |
| 45 | 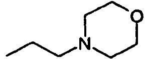 | 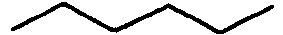 | 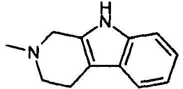 | 474 | R |
| 46 | 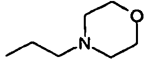 | 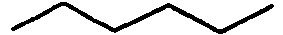 | 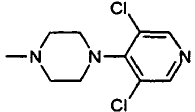 | 533 | R |
| 47 | 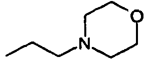 | 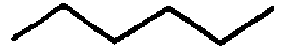 | 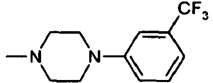 | 532 | R |
| 48 | 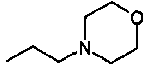 | 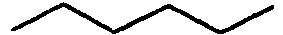 | 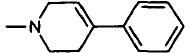 | 461 | R |
| 49 | 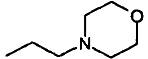 | 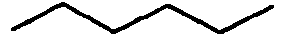 | 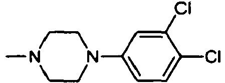 | 532 | R |
| 50 | 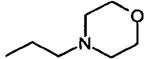 | 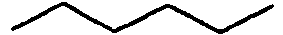 | 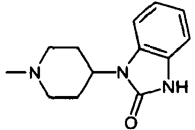 | 519 | R |
| 51 | 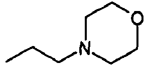 | 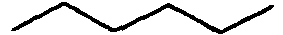 | 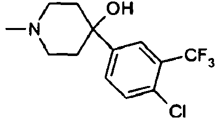 | 581 | R |
| 52 | 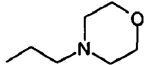 | 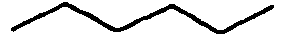 | 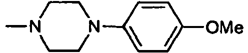 | 494 | R |
| 53 | 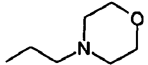 | 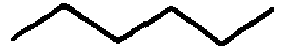 | 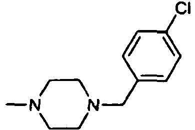 | 512 | R |
| 54 | 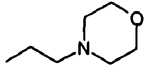 | 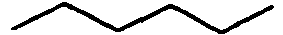 | 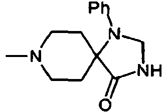 | 533 | R |
| 55 | 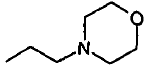 | 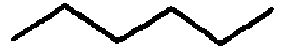 | 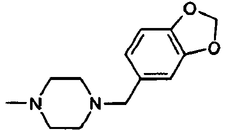 | 522 | R |
| 56 | 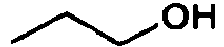 | 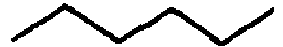 | 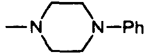 | 395 | R |
| 57 | 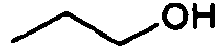 | 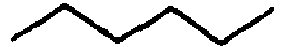 | 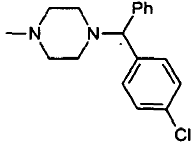 | 419 | R |
| 58 | 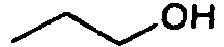 | 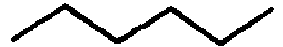 | 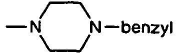 | 409 | R |
| 59 | 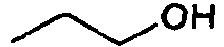 | 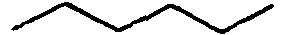 | 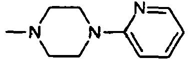 | 396 | R |
| 60 | 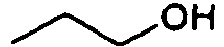 | 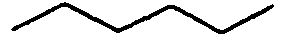 | 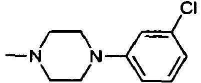 | 429 | R |
| 61 | 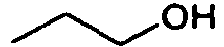 | 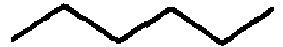 | 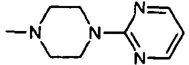 | 397 | R |
| 62 | 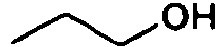 | 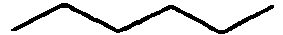 | 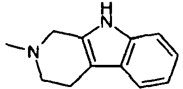 | 405 | R |
| 63 | 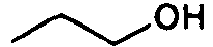 | 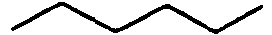 | 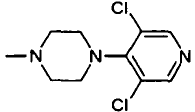 | 464 | R |
| 64 | 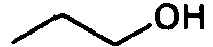 | 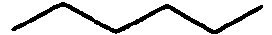 | 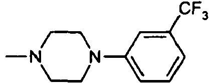 | 463 | R |
| 65 | 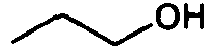 | 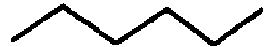 | 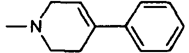 | 392 | R |
| 66 | 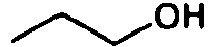 | 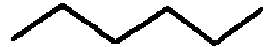 | 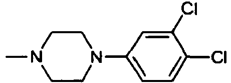 | 463 | R |
| 67 | 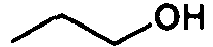 | 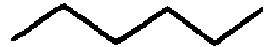 | 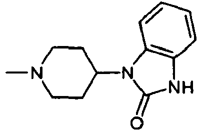 | 450 | R |
| 68 | 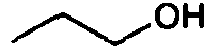 | 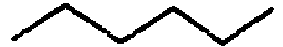 | 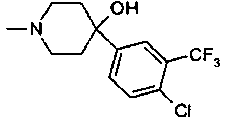 | 512 | R |
| 69 | 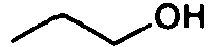 | 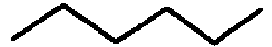 | 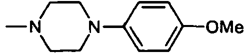 | 425 | R |
| 70 | 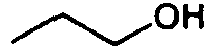 | 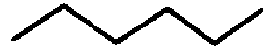 | 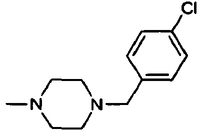 | 443 | R |
| 71 | 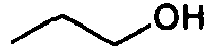 | 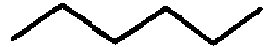 | 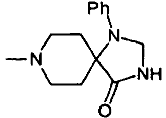 | 464 | R |
| 72 | 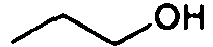 | 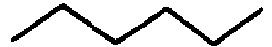 | 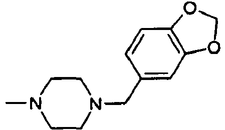 | 453 | R |
| 73 | 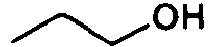 | 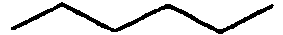 | 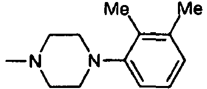 | 423 | R |
| 74 | 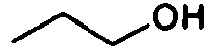 | 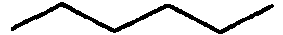 | 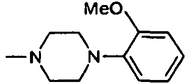 | 425 | R |
| 75 | 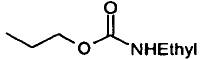 | 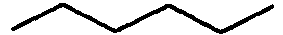 | 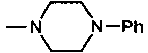 | 466 | I |
| 76 | 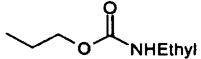 | 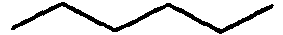 | 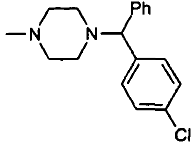 | 590 | I |
| 77 | 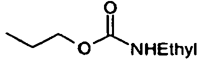 | 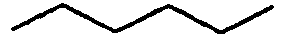 | 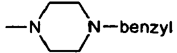 | 480 | I |
| 78 | 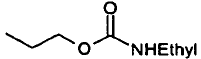 | 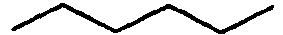 | 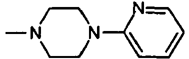 | 467 | I |
| 79 | 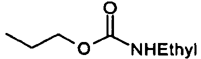 | 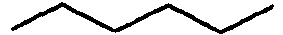 | 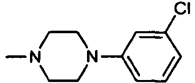 | 500 | I |
| 80 | 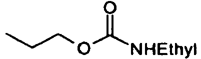 | 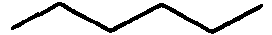 | 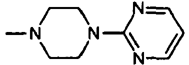 | 468 | I |
| 81 | 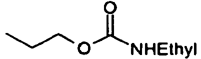 | 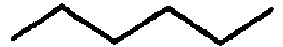 | 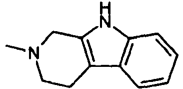 | 476 | I |
| 82 | 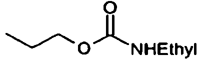 | 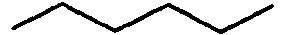 | 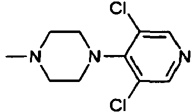 | 535 | I |
| 83 | 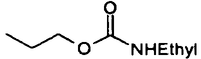 | 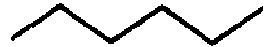 | 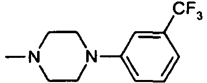 | 534 | I |
| 84 | 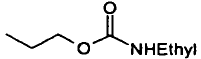 | 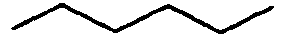 | 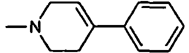 | 463 | I |
| 85 | 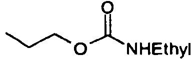 | 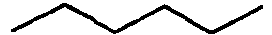 | 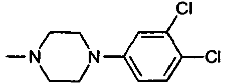 | 534 | I |
| 86 | 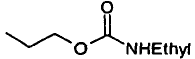 | 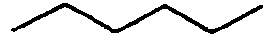 | 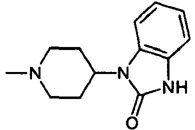 | 521 | I |
| 87 | 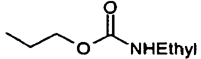 | 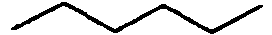 | 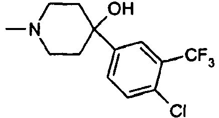 | 583 | I |
| 88 | 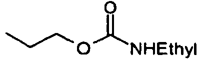 | 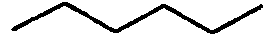 | 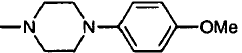 | 496 | I |
| 89 | 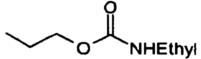 | 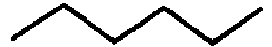 | 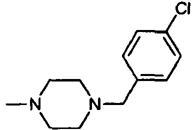 | 514 | I |
| 90 | 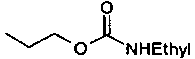 | 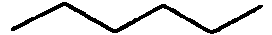 | 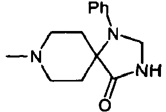 | 535 | I |
| 91 | 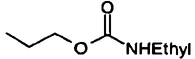 | 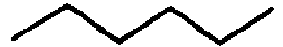 | 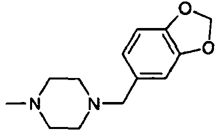 | 524 | I |
| 92 | 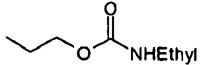 | 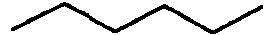 | 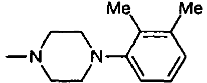 | 494 | I |
| 93 | 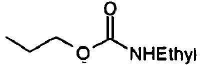 | 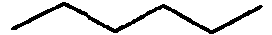 | 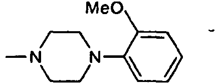 | 496 | I |
| 94 | 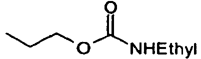 | 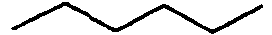 | 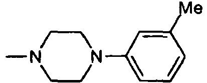 | 480 | I |
| 95 | 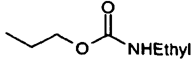 | 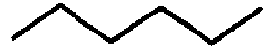 | 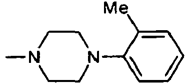 | 480 | I |
| 96 | 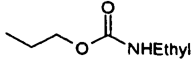 | 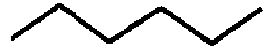 | 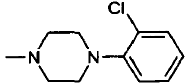 | 500 | I |
| 97 | 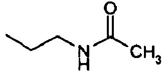 | 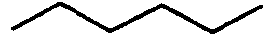 | 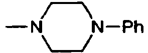 | 436 | I |
| 98 | 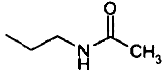 | 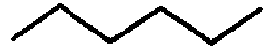 | 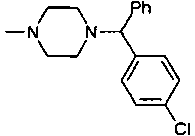 | 560 | I |
| 99 | 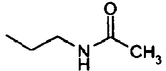 | 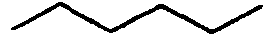 | 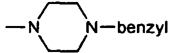 | 450 | I |
| 100 | 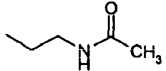 | 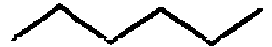 | 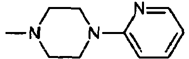 | 437 | I |
| 101 | 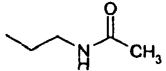 | 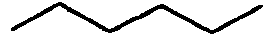 | 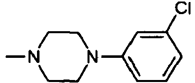 | 470 | I |
| 102 | 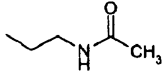 | 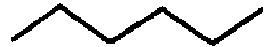 | 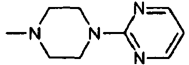 | 438 | I |
| 103 | 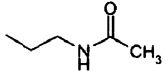 | 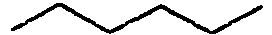 | 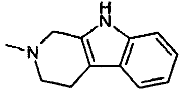 | 446 | I |
| 104 | 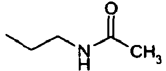 | 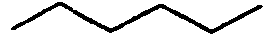 | 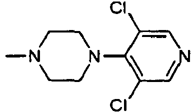 | 505 | I |
| 105 | 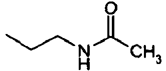 | 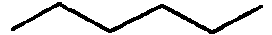 | 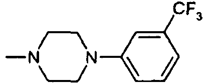 | 504 | I |
| 106 | 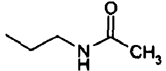 | 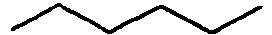 | 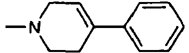 | 433 | I |
| 107 | 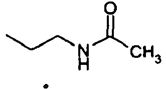 | 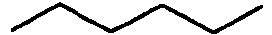 | 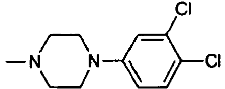 | 504 | I |
| 108 | 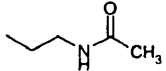 | 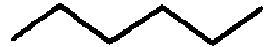 | 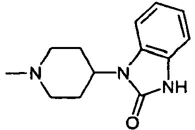 | 491 | I |
| 109 | 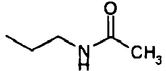 | 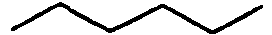 | 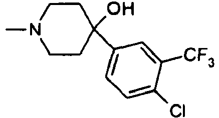 | 553 | I |
| 110 | 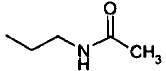 | 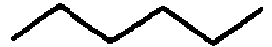 | 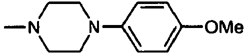 | 466 | I |
| 111 | 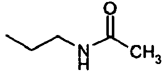 | 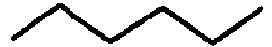 | 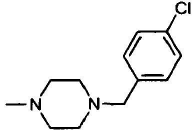 | 484 | I |
| 112 | 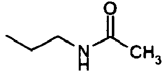 | 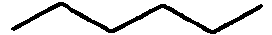 | 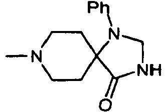 | 505 | I |
| 113 | 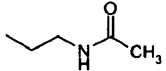 | 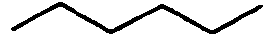 | 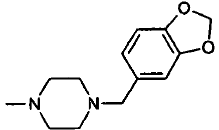 | 494 | I |
| 114 | 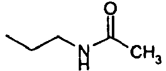 | 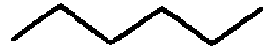 | 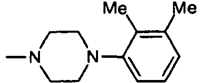 | 464 | I |
| 115 | 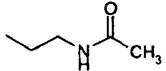 | 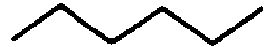 | 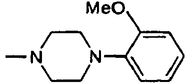 | 466 | I |
| 116 | 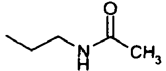 | 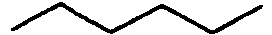 | 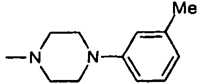 | 450 | I |
| 117 | 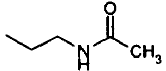 | 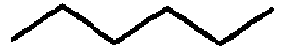 | 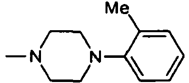 | 450 | I |
| 118 | 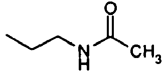 | 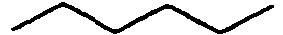 | 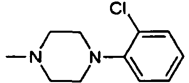 | 470 | I |
| 119 | 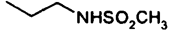 | 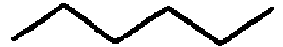 | 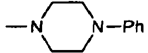 | 472 | I |
| 120 | 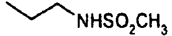 | 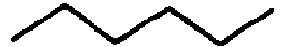 | 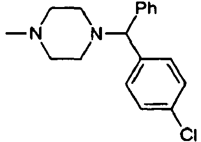 | 596 | I |
| 121 | 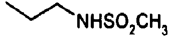 | 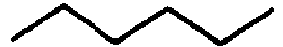 | 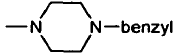 | 486 | I |
| 122 | 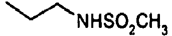 | 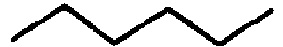 | 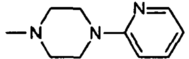 | 473 | I |
| 123 | 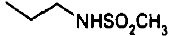 | 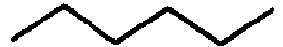 | 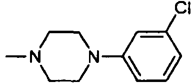 | 506 | I |
| 124 | 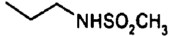 | 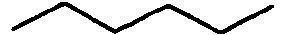 | 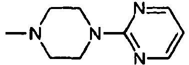 | 474 | I |
| 125 | 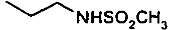 | 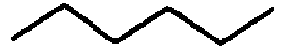 | 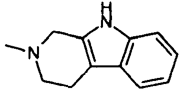 | 482 | I |
| 126 | 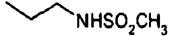 | 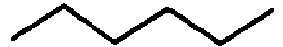 | 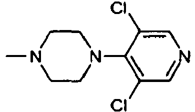 | 541 | I |
| 127 | 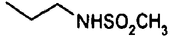 | 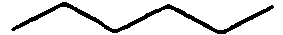 | 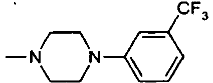 | 540 | I |
| 128 | 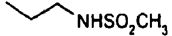 | 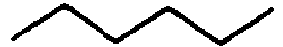 | 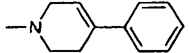 | 469 | I |
| 129 | 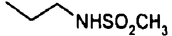 | 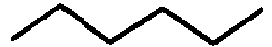 | 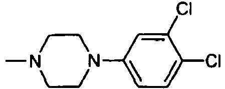 | 540 | I |
| 130 | 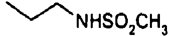 | 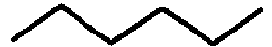 | 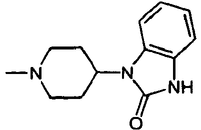 | 527 | I |
| 131 | 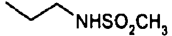 | 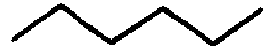 | 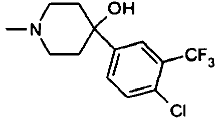 | 589 | I |
| 132 | 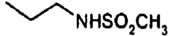 | 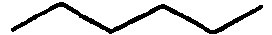 | 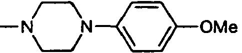 - | 502 | I |
| 133 | 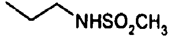 | 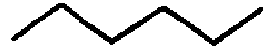 | 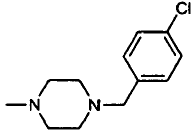 | 520 | I |
| 134 | 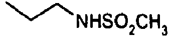 | 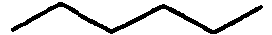 | 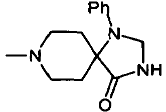 | 541 | I |
| 135 | 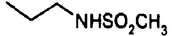 | 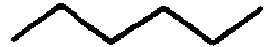 | 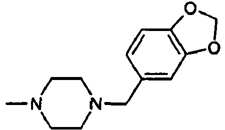 | 530 | I |
| 136 | 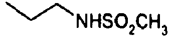 | 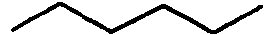 | 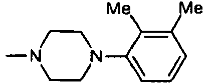 | 500 | I |
| 137 | 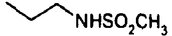 | 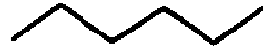 | 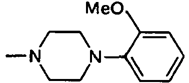 | 502 | I |
| 138 | 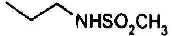 | 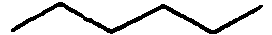 | 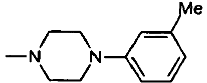 | 486 | I |
| 139 | 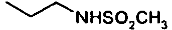 | 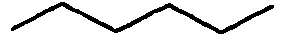 | 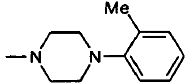 | 486 | I |
| 140 | 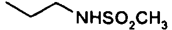 | 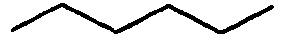 | 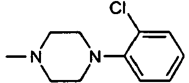 | 506 | I |
| 141 | 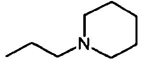 | 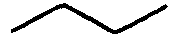 | 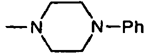 | 434 | R |
| 142 | 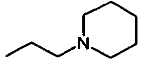 | 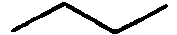 | 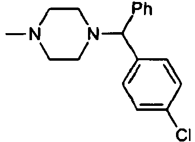 | 558 | R |
| 143 | 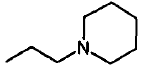 | 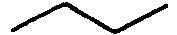 | 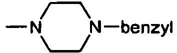 | 448 | R |
| 144 | 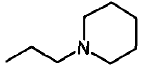 | 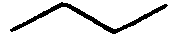 | 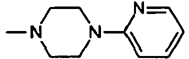 | 435 | R |
| 145 | 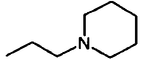 | 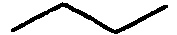 | 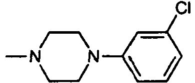 | 468 | R |
| 146 | 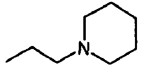 | 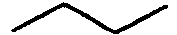 | 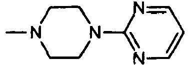 | 436 | R |
| 147 | 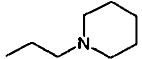 | 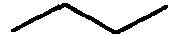 | 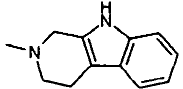 | 444 | R |
| 148 | 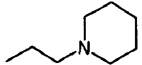 | 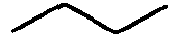 | 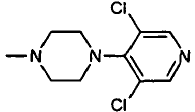 | 503 | R |
| 149 | 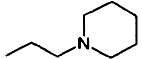 | 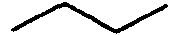 | 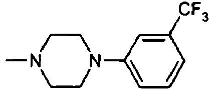 | 502 | R |
| 150 | 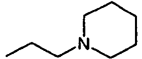 | 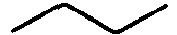 | 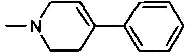 | 431 | R |
| 151 | 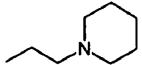 | 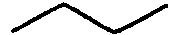 | 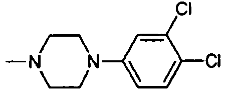 | 502 | R |
| 152 | 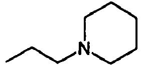 | 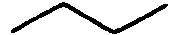 | 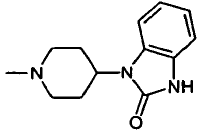 | 489 | R |
| 153 | 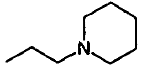 | 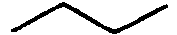 | 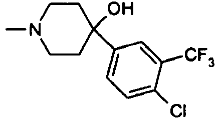 | 551 | R |
| 154 | 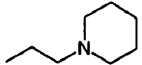 | 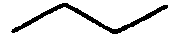 | 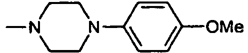 | 464 | R |
| 155 | 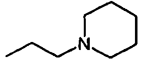 | 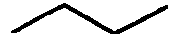 | 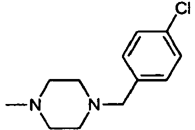 | 482 | R |
| 156 | 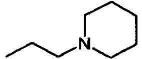 | 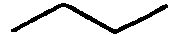 | 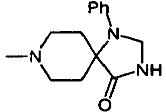 | 503 | R |
| 157 | 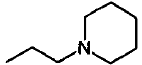 | 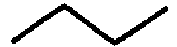 | 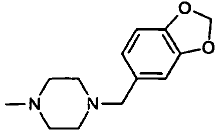 | 492 | R |
| 158 | 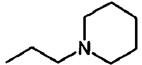 | 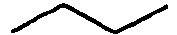 | 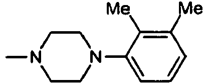 | 462 | R |
| 159 | 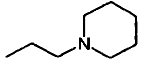 | 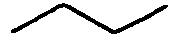 | 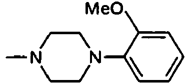 | 464 | R |
| 160 | 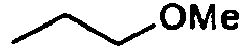 | 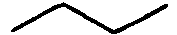 | 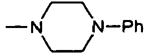 | 381 | I |
| 161 | 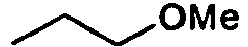 | 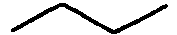 | 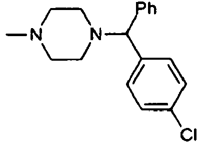 | 505 | I |
| 162 | 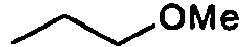 | 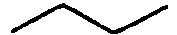 | 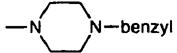 | 395 | I |
| 163 | 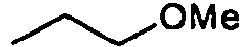 | 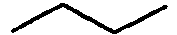 | 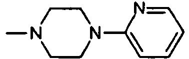 | 382 | I |
| 164 | 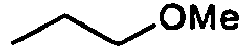 | 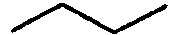 | 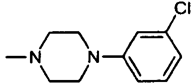 | 415 | I |
| 165 | 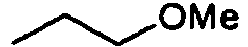 | 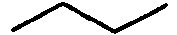 | 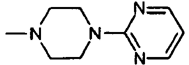 | 383 | I |
| 166 | 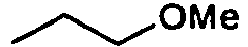 | 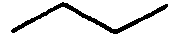 | 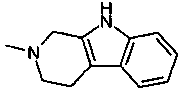 | 391 | I |
| 167 | 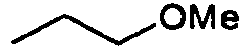 | 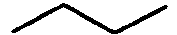 | 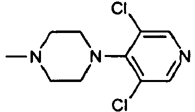 | 450 | I |
| 168 | 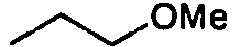 | 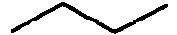 | 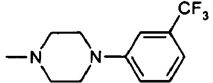 | 449 | I |
| 169 | 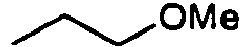 | 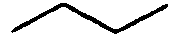 | 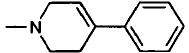 | 378 | I |
| 170 | 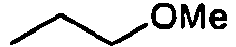 | 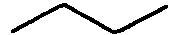 | 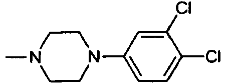 | 449 | I |
| 171 | 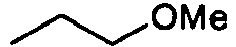 | 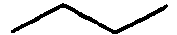 | 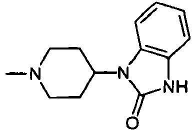 | 436 | I |
| 172 | 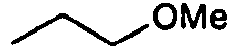 | 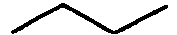 | 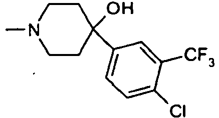 | 526 | I |
| 173 | 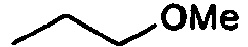 | 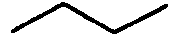 | 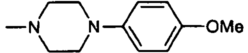 | 411 | I |
| 174 | 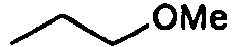 | 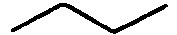 | 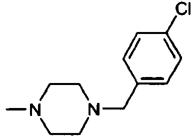 | 429 | I |
| 175 | 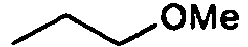 | 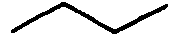 | 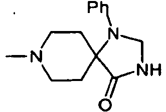 | 450 | I |
| 176 | 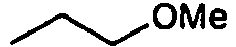 | 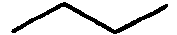 | 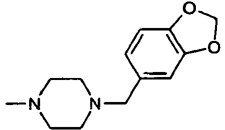 | 450 | I |
| 177 | 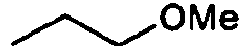 | 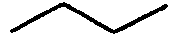 | 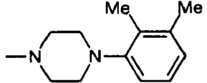 | 409 | I |
| 178 | 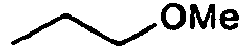 | 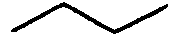 | 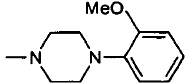 | 411 | I |
| 179 | 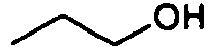 | 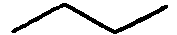 | 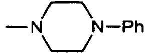 | 367 | R |
| 180 | 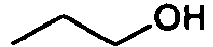 | 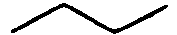 | 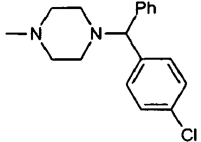 | 491 | R |
| 181 | 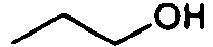 | 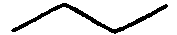 | 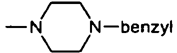 | 381 | R |
| 182 | 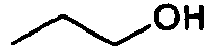 | 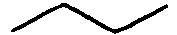 | 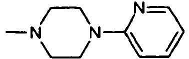 | 368 | R |
| 183 | 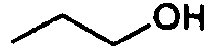 | 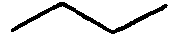 | 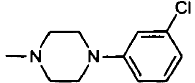 | 401 | R |
| 184 | 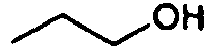 | 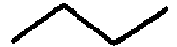 | 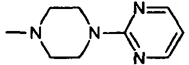 | 369 | R |
| 185 | 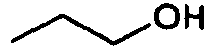 | 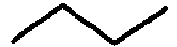 | 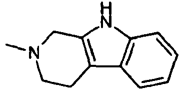 | 377 | R |
| 186 | 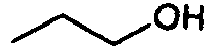 | 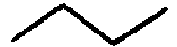 | 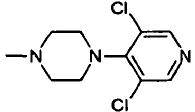 | 436 | R |
| 187 | 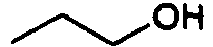 | 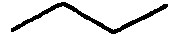 | 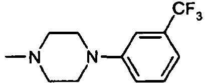 | 435 | R |
| 188 | 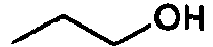 | 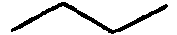 | 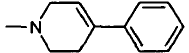 | 364 | R |
| 189 | 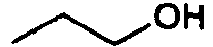 | 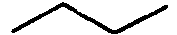 | 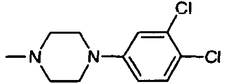 | 435 | R |
| 190 | 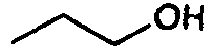 | 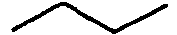 | 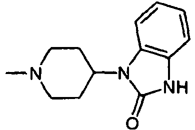 | 422 | R |
| 191 | 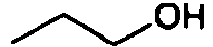 | 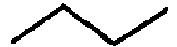 | 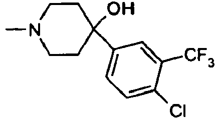 | 484 | R |
| 192 | 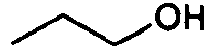 | 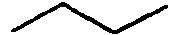 | 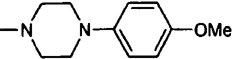 - | 397 | R |
| 193 | 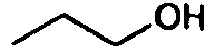 | 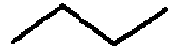 | 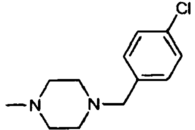 | 415 | R |
| 194 | 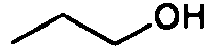 | 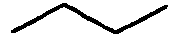 | 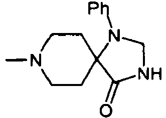 | 436 | R |
| 195 | 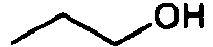 | 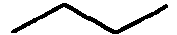 | 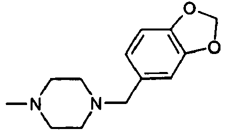 | 436 | R |
| 196 | 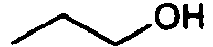 | 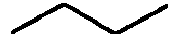 | 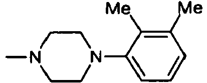 | 395 | R |
| 197 | 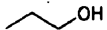 | 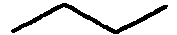 | 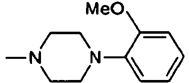 | 397 | R |
| 198 | 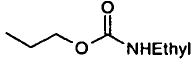 | 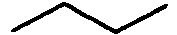 | 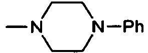 | 438 | I |
| 199 | 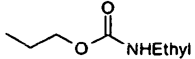 | 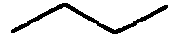 | 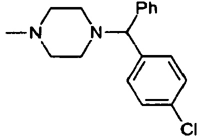 | 562 | I |
| 200 | 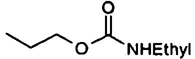 | 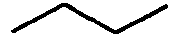 | 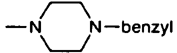 | 452 | I |
| 201 | 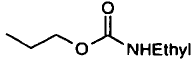 | 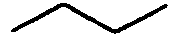 | 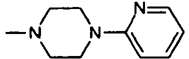 | 439 | I |
| 202 | 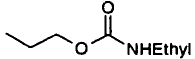 | 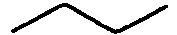 | 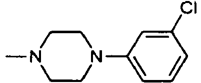 | 472 | I |
| 203 | 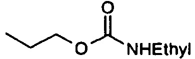 | 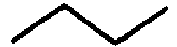 | 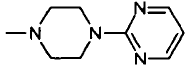 | 440 | I |
| 204 | 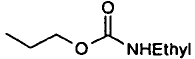 | 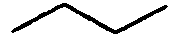 | 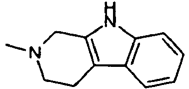 | 448 | I |
| 205 | 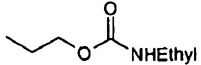 | 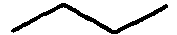 | 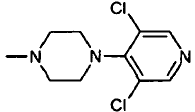 | 507 | I |
| 206 | 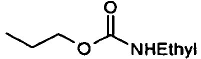 | 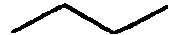 | 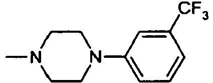 | 506 | I |
| 207 | 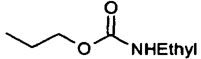 | 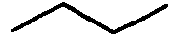 | 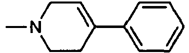 | 435 | I |
| 208 | 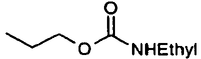 | 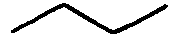 | 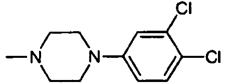 | 506 | I |
| 209 | 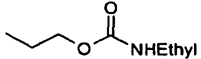 | 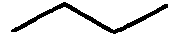 | 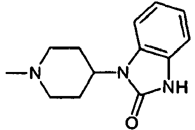 | 493 | I |
| 210 | 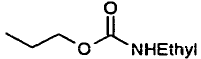 | 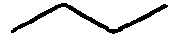 | 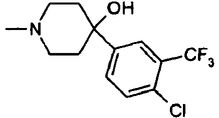 | 555 | I |
| 211 | 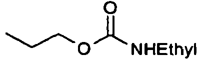 | 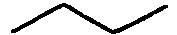 | 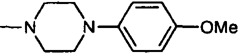 - | 468 | I |
| 212 | 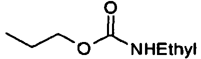 | 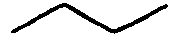 | 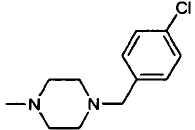 | 486 | I |
| 213 | 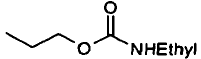 | 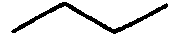 | 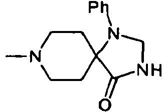 | 507 | I |
| 214 | 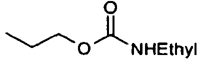 | 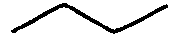 | 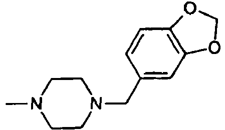 | 496 | I |
| 215 | 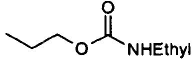 | 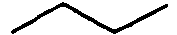 | 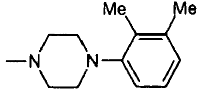 | 466 | I |
| 216 | 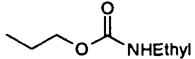 | 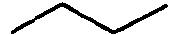 | 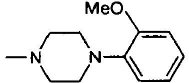 | 468 | I |
| 217 | 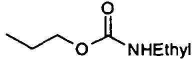 | 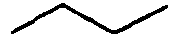 | 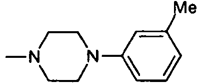 | 452 | I |
| 218 | 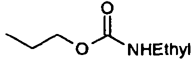 | 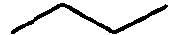 | 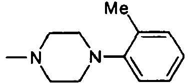 | 452 | I |
| 219 | 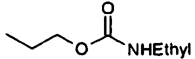 | 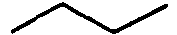 | 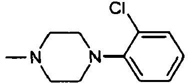 | 472 | I |
| 220 | 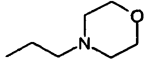 | 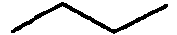 | 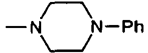 | 436 | R |
| 221 | 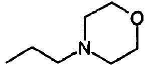 | 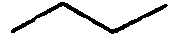 | 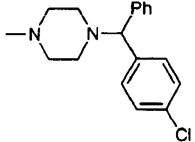 | 560 | R |
| 222 | 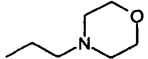 | 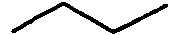 | 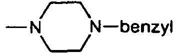 | 450 | R |
| 223 | 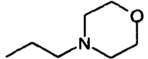 | 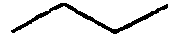 | 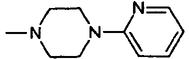 | 437 | R |
| 224 | 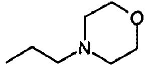 | 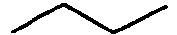 | 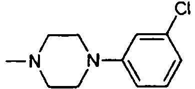 | 470 | R |
| 225 | 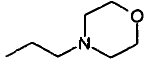 | 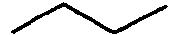 | 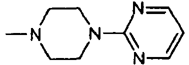 | 438 | R |
| 226 | 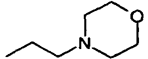 | 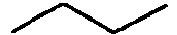 | 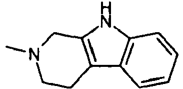 | 446 | R |
| 227 | 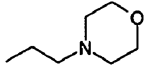 | 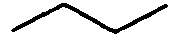 | 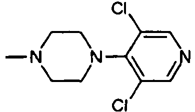 | 533 | R |
| 228 | 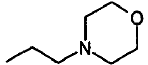 | 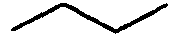 | 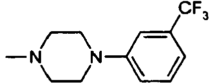 | 504 | R |
| 229 | 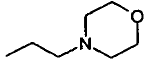 | 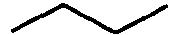 | 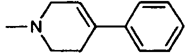 | 433 | R |
| 230 | 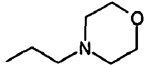 | 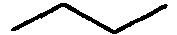 | 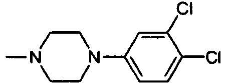 | 504 | R |
| 231 | 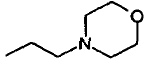 | 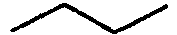 | 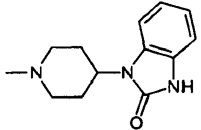 | 491 | R |
| 232 | 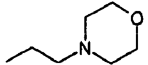 | 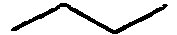 | 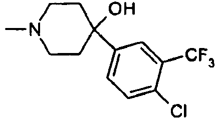 | 553 | R |
| 233 | 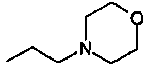 | 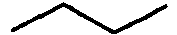 | 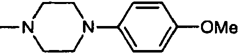 - | 466 | R |
| 234 | 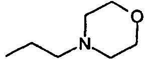 | 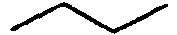 | 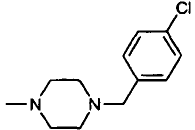 | 484 | R |
| 235 | 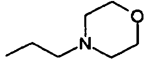 | 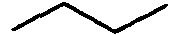 | 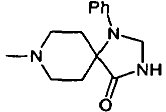 | 505 | R |
| 236 | 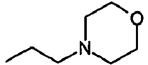 | 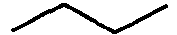 | 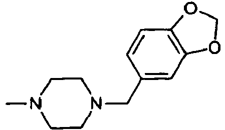 | 494 | R |
| 237 | 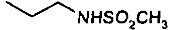 | 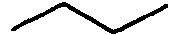 | 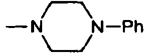 | 444 | I |
| 238 | 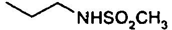 | 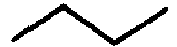 | 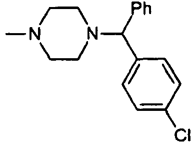 | 568 | I |
| 239 | 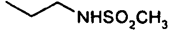 | 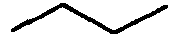 | 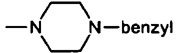 | 458 | I |
| 240 | 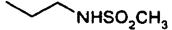 | 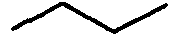 | 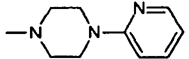 | 445 | I |
| 241 | 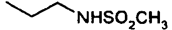 | 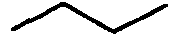 | 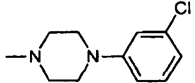 | 478 | I |
| 242 | 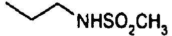 | 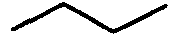 | 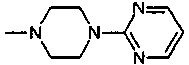 | 446 | I |
| 243 | 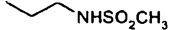 | 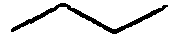 | 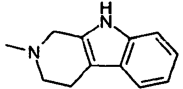 | 454 | I |
| 244 | 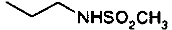 | 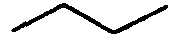 | 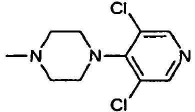 | 513 | I |
| 245 | 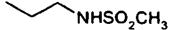 | 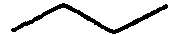 | 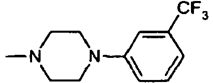 | 512 | I |
| 246 | 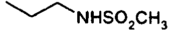 | 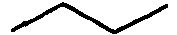 | 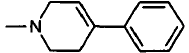 | 441 | I |
| 247 | 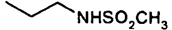 | 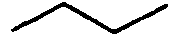 | 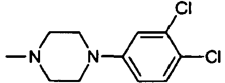 | 512 | I |
| 248 | 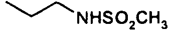 | 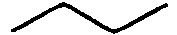 | 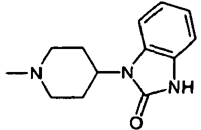 | 499 | I |
| 249 | 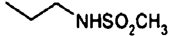 | 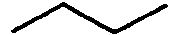 | 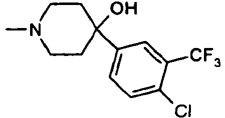 | 561 | I |
| 250 | 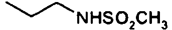 | 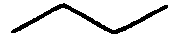 | 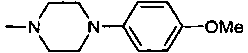 | 474 | I |
| 251 | 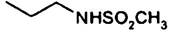 | 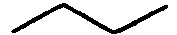 | 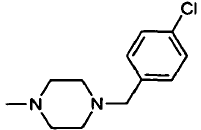 | 492 | I |
| 252 | 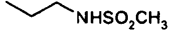 | 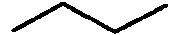 | 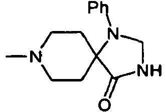 | 513 | I |
| 253 | 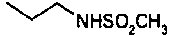 | 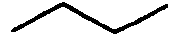 | 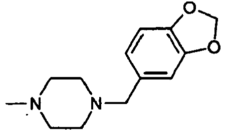 | 502 | I |
| 254 | 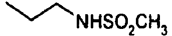 | 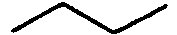 | 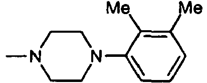 | 472 | I |
| 255 | 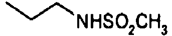 | 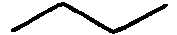 | 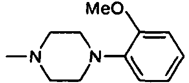 | 474 | I |
| 256 | 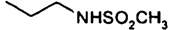 | 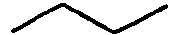 | 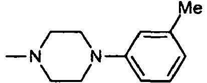 | 458 | I |
| 257 | 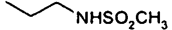 | 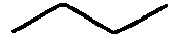 | 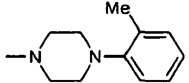 | 458 | I |
| 258 | 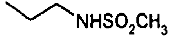 | 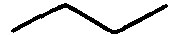 | 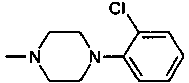 | 478 | I |
| 259 | 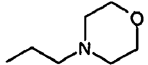 | 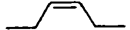 | 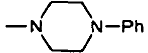 | 462 | R |
| 260 | 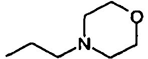 | 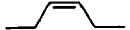 | 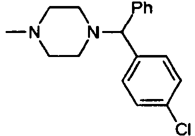 | 586 | R |
| 261 | 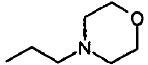 | 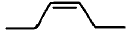 | 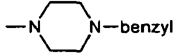 | 476 | R |
| 262 | 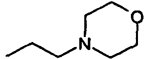 | 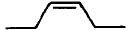 | 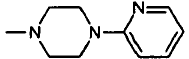 | 463 | R |
| 263 | 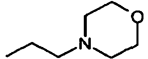 | 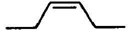 | 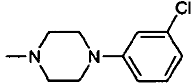 | 496 | R |
| 264 | 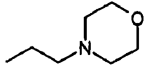 | 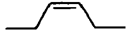 | 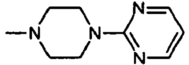 | 464 | R |
| 265 | 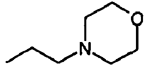 | 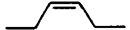 | 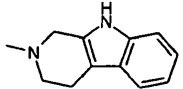 | 472 | R |
| 266 | 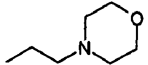 | 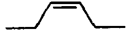 | 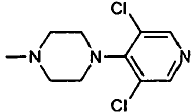 | 531 | R |
| 267 | 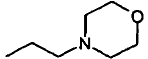 | 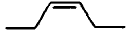 | 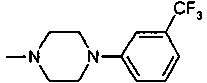 | 530 | R |
| 268 | 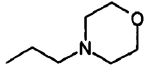 | 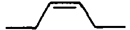 | 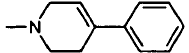 | 459 | R |
| 269 | 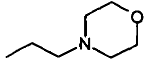 | 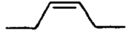 | 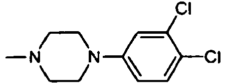 | 530 | R |
| 270 | 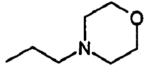 | 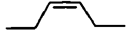 | 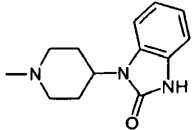 | 517 | R |
| 271 | 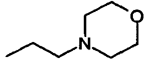 | 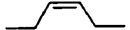 | 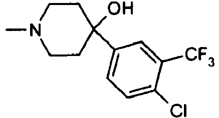 | 579 | R |
| 272 | 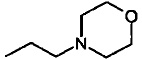 | 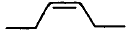 | 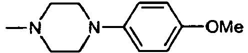 | 492 | R |
| 273 | 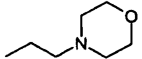 | 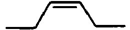 | 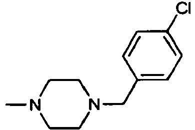 | 510 | R |
| 274 | 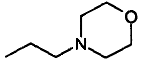 | 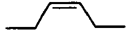 | 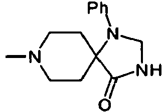 | 531 | R |
| 275 | 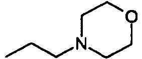 | 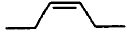 | 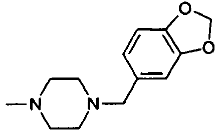 | 520 | R |
| 276 | 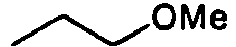 | 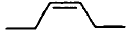 | 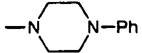 | 407 | I |
| 277 | 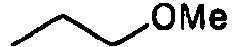 | 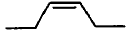 | 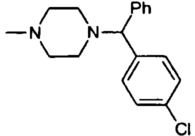 | 531 | I |
| 278 | 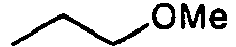 | 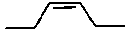 | 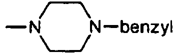 | 421 | I |
| 279 | 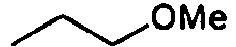 | 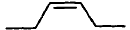 | 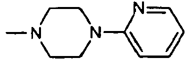 | 408 | I |
| 280 | 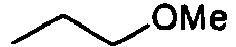 | 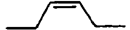 | 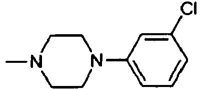 | 441 | I |
| 281 | 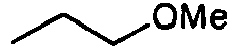 | 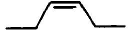 | 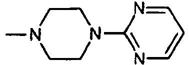 | 409 | I |
| 282 | 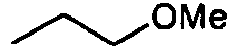 | 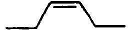 | 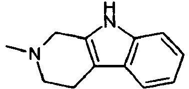 | 417 | I |
| 283 | 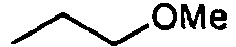 | 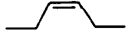 | 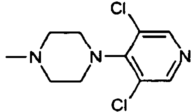 | 476 | I |
| 284 | 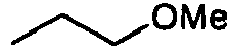 | 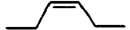 | 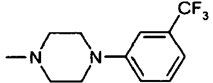 | 475 | I |
| 285 | 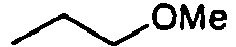 | 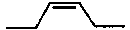 | 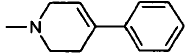 | 404 | I |
| 286 | 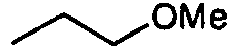 | 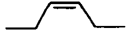 | 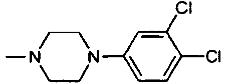 | 475 | I |
| 287 | 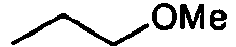 | 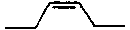 | 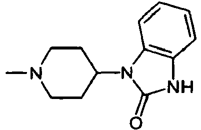 | 462 | I |
| 288 | 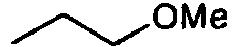 | 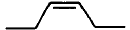 | 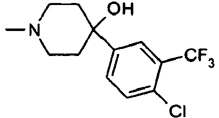 | 524 | I |
| 289 | 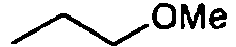 | 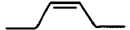 | 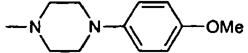 | 437 | I |
| 290 | 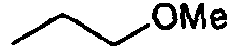 | 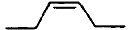 | 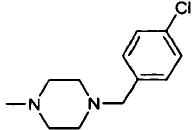 | 455 | I |
| 291 | 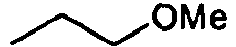 | 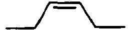 | 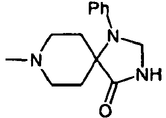 | 476 | I |
| 292 | 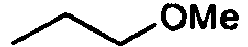 | 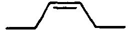 | 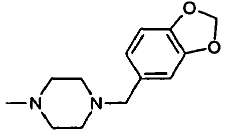 | 465 | I |
| 293 | 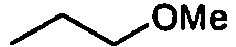 | 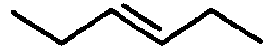 | 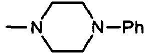 | 407 | I |
| 294 | 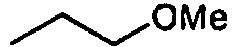 | 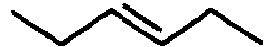 | 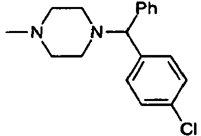 | 531 | I |
| 295 | 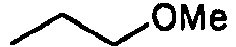 | 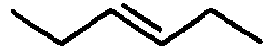 | 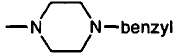 | 421 | I |
| 296 | 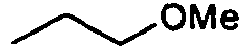 | 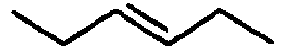 | 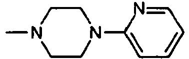 | 408 | I |
| 297 | 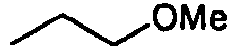 | 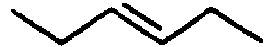 | 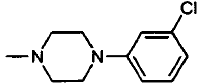 | 441 | I |
| 298 | 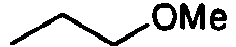 | 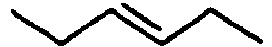 | 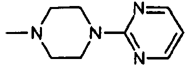 | 409 | I |
| 299 | 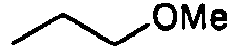 | 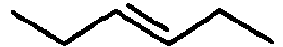 | 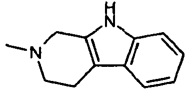 | 417 | I |
| 300 | 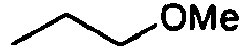 | 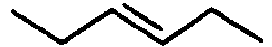 | 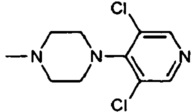 | 432 | I |
| 301 | 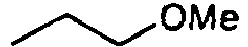 | 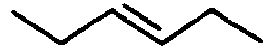 | 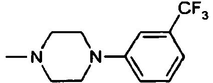 | 475 | I |
| 302 | 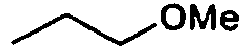 | 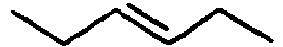 | 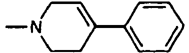 | 404 | I |
| 303 | 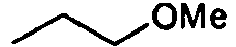 | 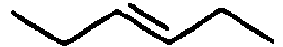 | 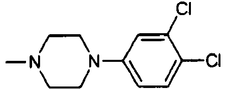 | 475 | I |
| 304 | 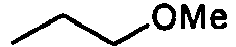 | 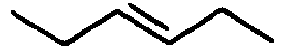 | 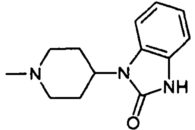 | 462 | I |
| 305 | 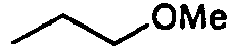 | 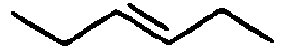 | 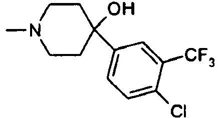 | 524 | I |
| 306 | 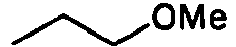 | 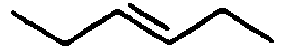 | 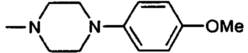 | 437 | I |
| 307 | 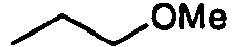 | 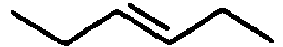 | 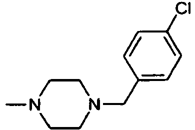 | 455 | I |
| 308 | 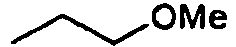 | 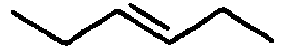 | 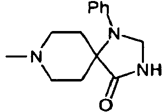 | 476 | I |
| 309 | 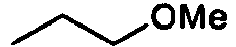 | 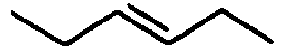 | 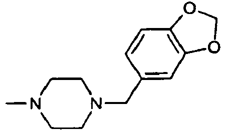 | 465 | I |
| 310 | 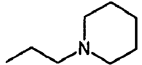 | 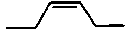 | 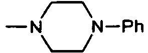 | 460 | R |
| 311 | 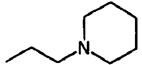 | 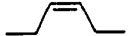 | 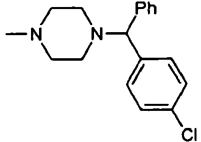 | 584 | R |
| 312 | 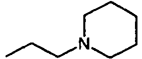 | 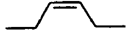 | 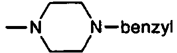 | 474 | R |
| 313 | 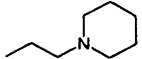 | 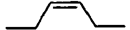 | 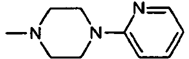 | 461 | R |
| 314 | 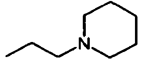 | 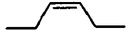 | 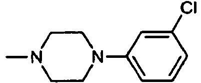 | 694 | R |
| 315 | 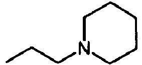 | 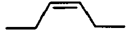 | 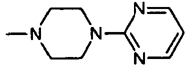 | 462 | R |
| 316 | 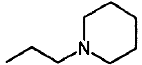 | 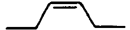 | 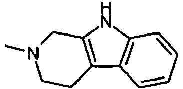 | 470 | R |
| 317 | 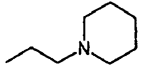 | 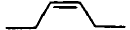 | 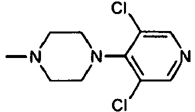 | 465 | R |
| 318 | 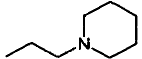 | 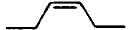 | 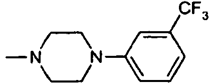 | 528 | R |
| 319 | 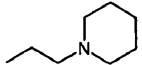 | 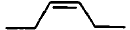 | 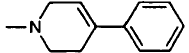 | 457 | R |
| 320 | 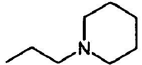 | 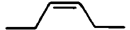 | 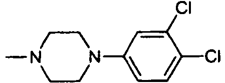 | 528 | R |
| 321 | 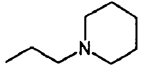 | 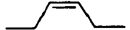 | 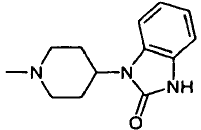 | 515 | R |
| 322 | 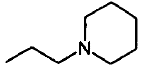 | 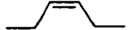 | 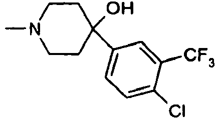 | 577 | R |
| 323 | 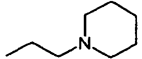 | 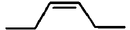 | 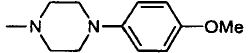 | 490 | R |
| 324 | 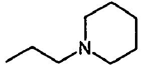 | 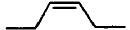 | 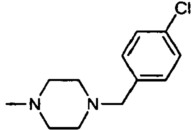 | 508 | R |
| 325 | 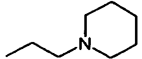 | 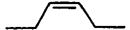 | 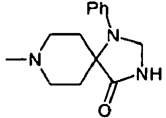 | 529 | R |
| 326 | 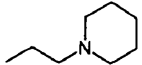 | 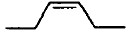 | 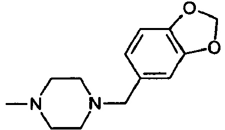 | 518 | R |
| 327 | 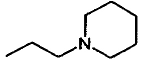 | 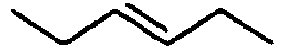 | 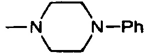 | 460 | R |
| 328 | 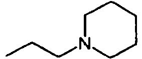 | 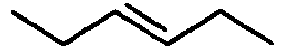 | 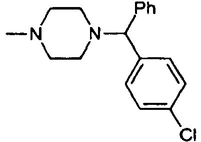 | 584 | R |
| 329 | 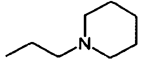 | 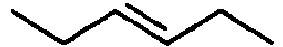 | 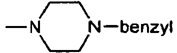 | 474 | R |
| 330 | 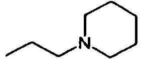 | 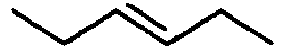 | 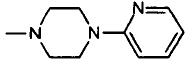 | 461 | R |
| 331 | 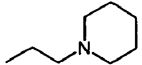 | 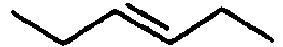 | 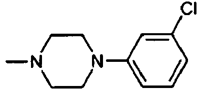 | 494 | R |
| 332 | 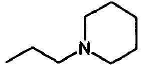 | 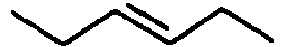 | 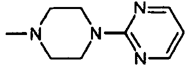 | 462 | R |
| 333 | 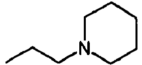 | 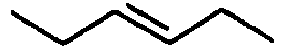 | 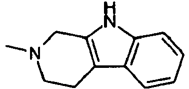 | 470 | R |
| 334 | 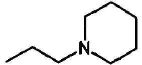 | 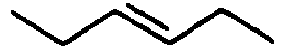 | 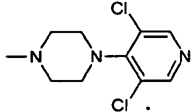 | 465 | R |
| 335 | 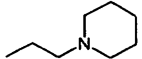 | 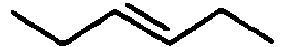 | 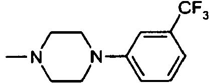 | 528 | R |
| 336 | 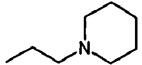 | 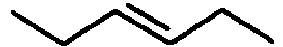 | 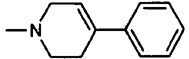 | 457 | R |
| 337 | 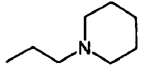 | 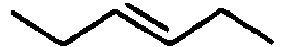 | 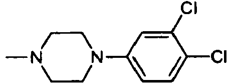 | 528 | R |
| 338 | 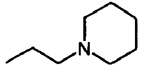 | 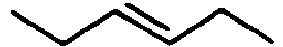 | 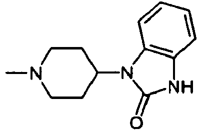 | 515 | R |
| 339 | 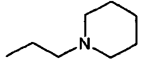 | 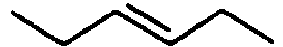 | 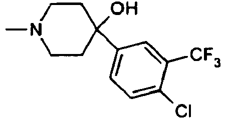 | 577 | R |
| 340 | 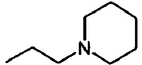 | 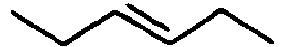 | 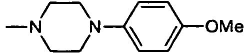 | 490 | R |
| 341 | 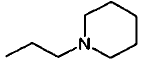 | 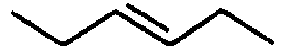 | 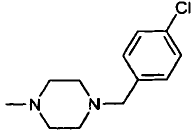 | 508 | R |
| 342 | 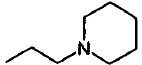 | 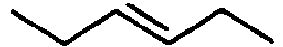 | 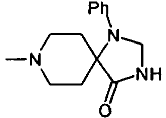 | 529 | R |
| 343 | 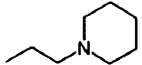 | 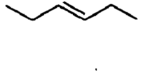 | 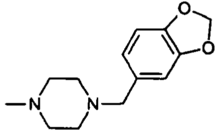 | 518 | R |
| 344 | 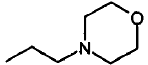 | 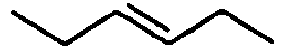 | 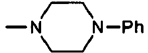 | 462 | R |
| 345 | 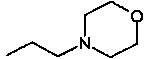 | 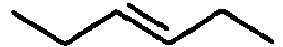 | 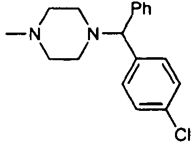 | 586 | R |
| 346 | 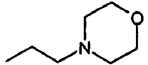 | 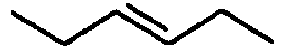 | 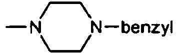 | 476 | R |
| 347 | 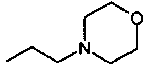 | 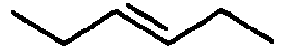 | 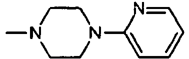 | 463 | R |
| 348 | 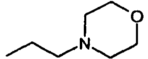 | 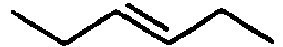 | 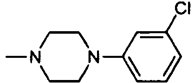 | 496 | R |
| 349 | 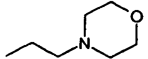 | 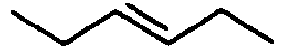 | 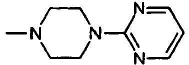 | 464 | R |
| 350 | 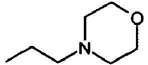 | 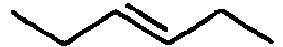 | 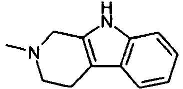 | 472 | R |
| 351 | 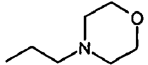 | 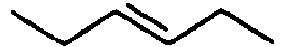 | 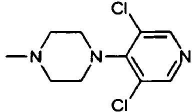 | 467 | R |
| 352 | 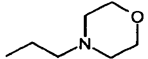 | 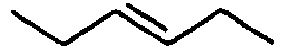 | 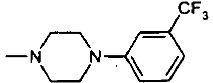 | 530 | R |
| 353 | 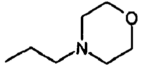 | 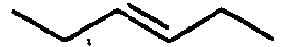 | 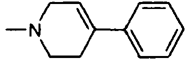 | 459 | R |
| 354 | 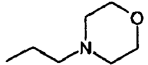 | 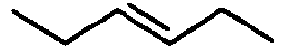 | 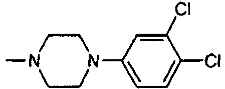 | 530 | R |
| 355 | 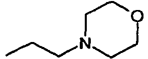 | 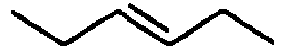 | 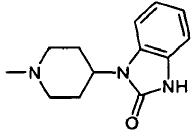 | 517 | R |
| 356 | 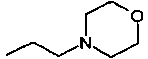 | 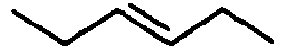 | 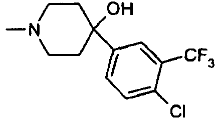 | 579 | R |
| 357 | 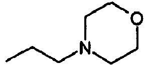 | 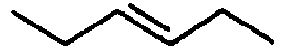 | 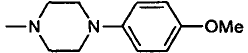 | 492 | R |
| 358 | 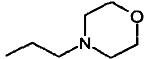 | 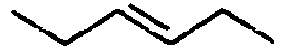 | 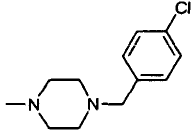 | 510 | R |
| 359 | 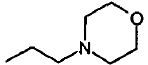 | 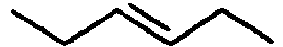 | 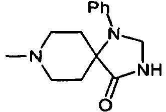 | 531 | R |
| 360 | 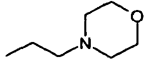 | 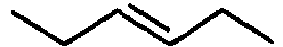 | 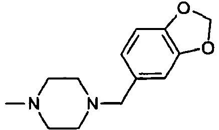 | 520 | R |
| 361 | 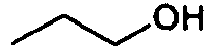 | 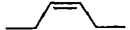 | 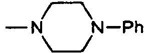 | 393 | R |
| 362 | 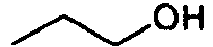 | 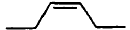 | 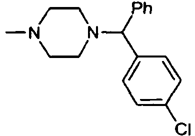 | 517 | R |
| 363 | 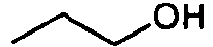 | 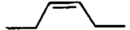 | 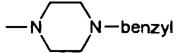 | 407 | R |
| 364 | 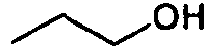 | 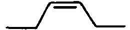 | 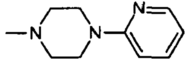 | 394 | R |
| 365 | 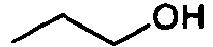 | 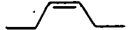 | 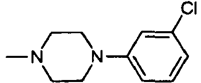 | 427 | R |
| 366 | 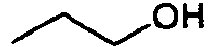 | 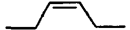 | 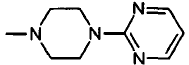 | 395 | R |
| 367 | 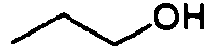 | 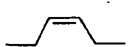 | 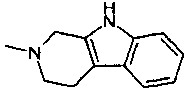 | 403 | R |
| 368 | 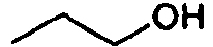 | 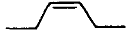 | 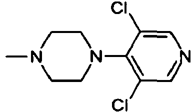 | 476 | R |
| 369 | 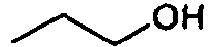 | 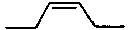 | 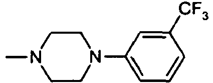 | 461 | R |
| 370 | 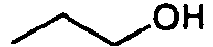 | 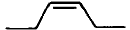 | 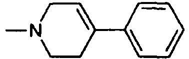 | 390 | R |
| 371 | 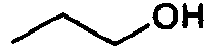 | 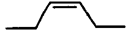 | 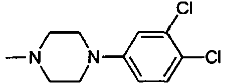 | 461 | R |
| 372 | 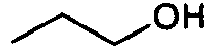 | 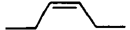 | 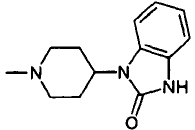 | 448 | R |
| 373 | 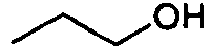 | 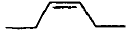 | 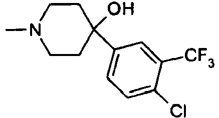 | 510 | R |
| 374 | 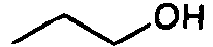 | 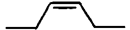 | 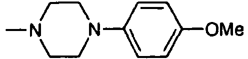 | 423 | R |
| 375 | 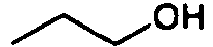 | 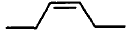 | 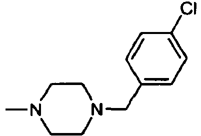 | 441 | R |
| 376 | 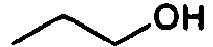 | 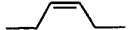 | 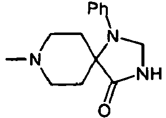 | 462 | R |
| 377 | 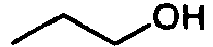 | 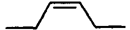 | 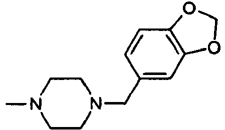 | 451 | R |
| 378 | 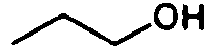 | 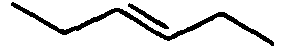 | 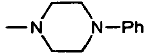 | 393 | R |
| 379 | 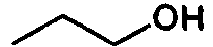 | 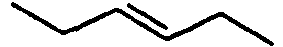 | 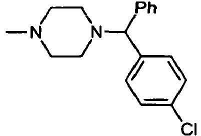 | 517 | R |
| 380 | 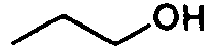 | 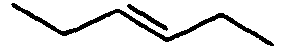 | 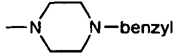 | 407 | R |
| 381 | 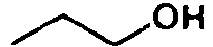 | 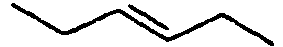 | 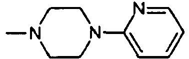 | 394 | R |
| 382 | 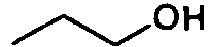 | 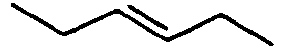 | 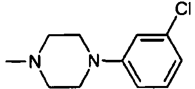 | 427 | R |
| 383 | 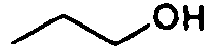 | 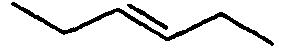 | 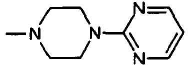 | 395 | R |
| 384 | 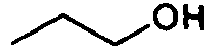 | 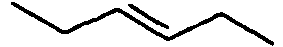 | 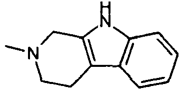 | 403 | R |
| 385 | 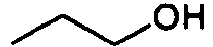 | 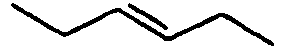 | 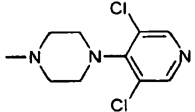 | 476 | R |
| 386 | 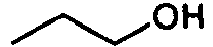 | 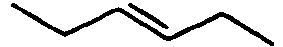 | 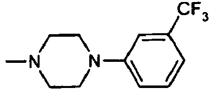 | 461 | R |
| 387 | 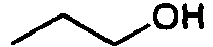 | 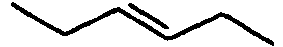 | 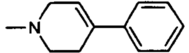 | 390 | R |
| 388 | 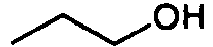 | 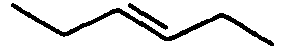 | 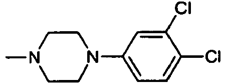 | 461 | R |
| 389 | 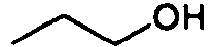 | 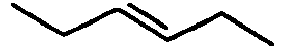 | 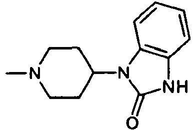 | 448 | R |
| 390 | 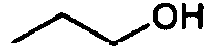 | 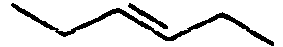 | 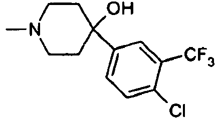 | 510 | R |
| 391 | 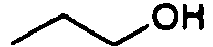 | 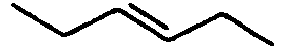 | 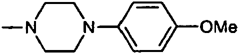 | 423 | R |
| 392 | 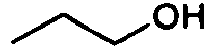 | 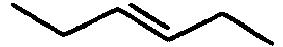 | 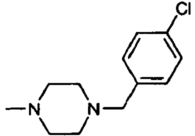 | 441 | R |
| 393 | 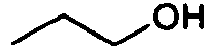 | 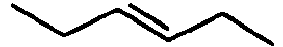 | 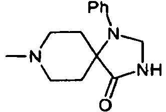 | 462 | R |
| 394 | 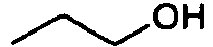 | 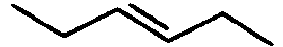 | 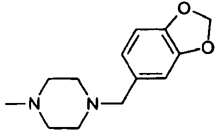 | 451 | R |
| 395 | 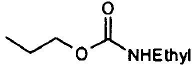 | 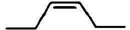 | 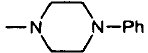 | 464 | I |
| 396 | 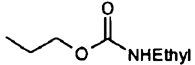 | 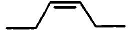 | 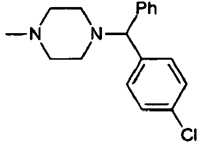 | 588 | I |
| 397 | 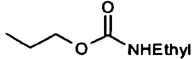 | 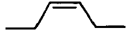 | 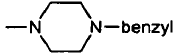 | 478 | I |
| 398 | 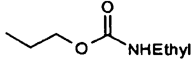 | 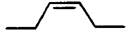 | 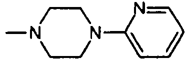 | 465 | I |
| 399 | 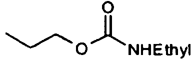 | 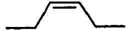 | 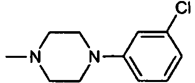 | 699 | I |
| 400 | 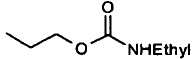 | 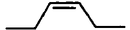 | 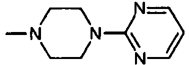 | 466 | I |
| 401 | 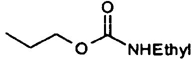 | 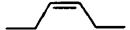 | 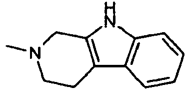 | 474 | I |
| 402 | 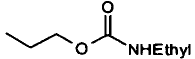 | 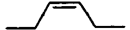 | 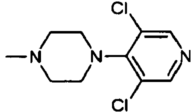 | 469 | I |
| 403 | 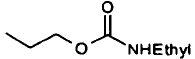 | 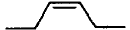 | 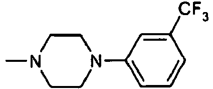 | 532 | I |
| 404 | 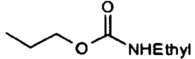 | 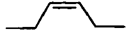 | 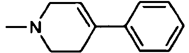 | 461 | I |
| 405 | 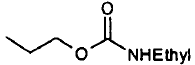 | 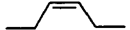 | 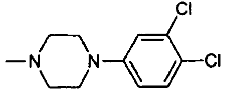 | 532 | I |
| 406 | 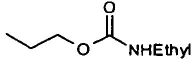 | 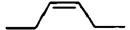 | 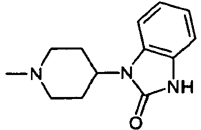 | 519 | I |
| 407 | 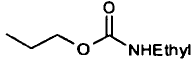 | 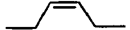 | 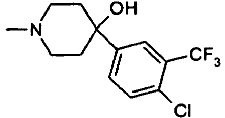 | 581 | I |
| 408 | 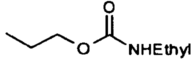 | 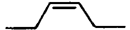 | 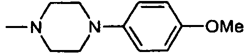 | 494 | I |
| 409 | 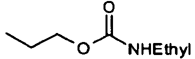 | 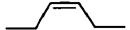 | 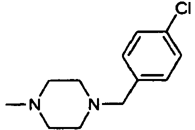 | 512 | I |
| 410 | 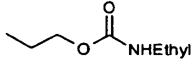 | 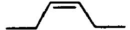 | 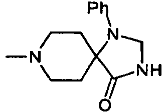 | 533 | I |
| 411 | 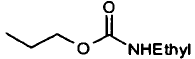 | 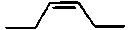 | 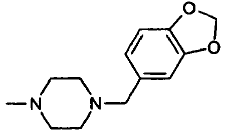 | 522 | I |
| 412 | 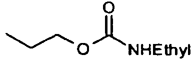 | 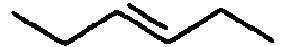 | 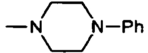 | 464 | I |
| 413 | 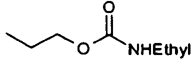 | 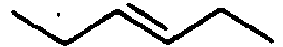 | 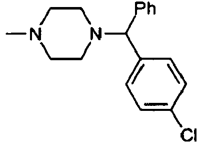 | 588 | I |
| 414 | 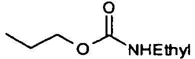 | 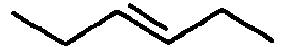 | 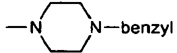 | 478 | I |
| 415 | 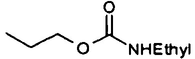 | 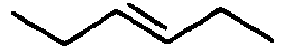 | 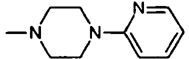 | 465 | I |
| 416 | 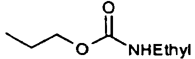 | 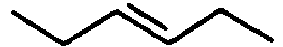 | 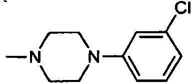 | 498 | I |
| 417 | 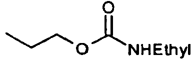 | 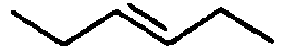 | 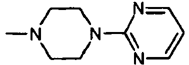 | 466 | I |
| 418 | 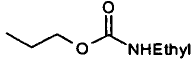 | 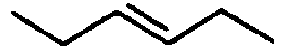 | 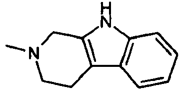 | 474 | I |
| 419 | 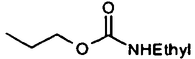 | 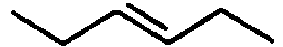 | 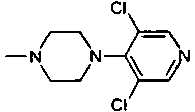 | 469 | I |
| 420 | 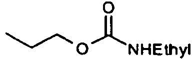 | 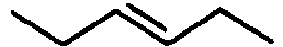 | 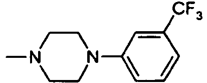 | 532 | I |
| 421 | 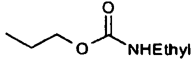 | 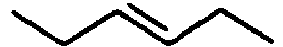 | 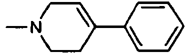 | 461 | I |
| 422 | 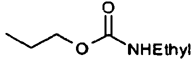 | 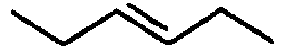 | 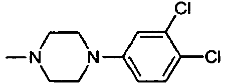 | 532 | I |
| 423 | 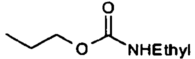 | 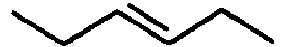 | 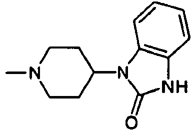 | 519 | I |
| 424 | 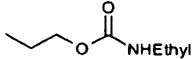 | 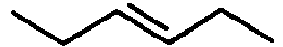 | 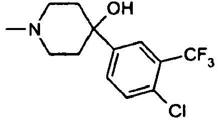 | 581 | I |
| 425 | 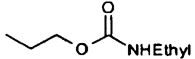 | 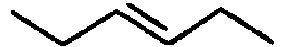 | 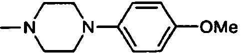 - | 494 | I |
| 426 | 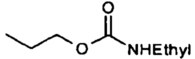 | 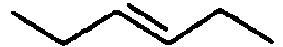 | 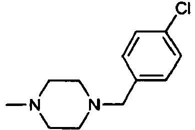 | 512 | I |
| 427 | 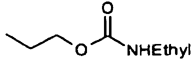 | 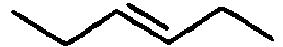 | 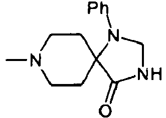 | 533 | I |
| 428 | 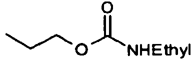 | 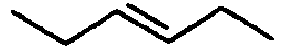 | 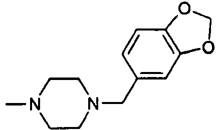 | 522 | I |
| 429 | 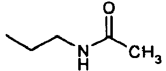 | 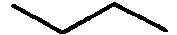 | 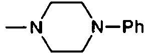 | 408 | I |
| 430 | 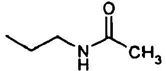 | 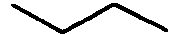 | 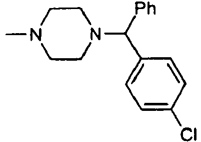 | 532 | I |
| 431 | 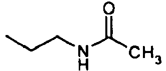 | 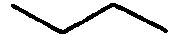 | 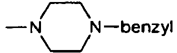 | 422 | I |
| 432 | 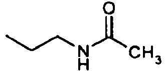 | 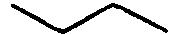 | 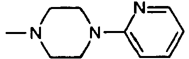 | 409 | I |
| 433 | 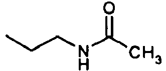 | 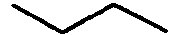 | 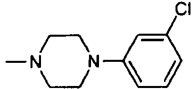 | 442 | I |
| 434 | 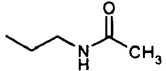 | 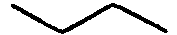 | 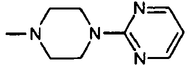 | 410 | I |
| 435 | 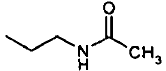 | 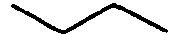 | 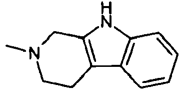 | 418 | I |
| 436 | 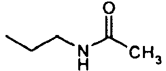 | 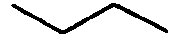 | 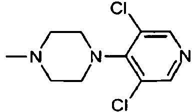 | 477 | I |
| 437 | 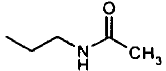 | 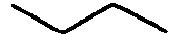 | 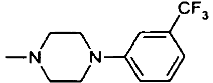 | 476 | I |
| 438 | 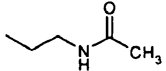 | 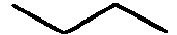 | 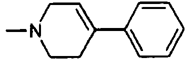 | 405 | I |
| 439 | 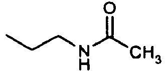 | 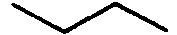 | 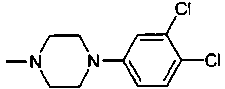 | 476 | I |
| 440 | 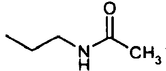 | 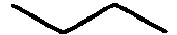 | 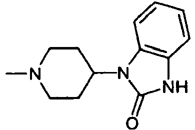 | 463 | I |
| 441 | 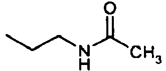 | 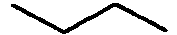 | 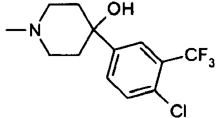 | 525 | I |
| 442 | 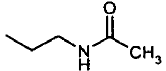 | 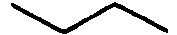 | 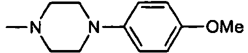 | 438 | I |
| 443 | 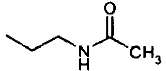 | 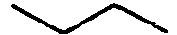 | 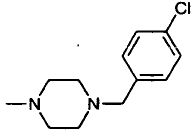 | 456 | I |
| 444 | 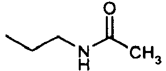 | 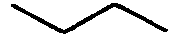 | 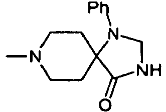 | 477 | I |
| 445 | 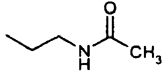 | 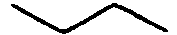 | 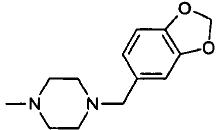 | 466 | I |
| 446 | 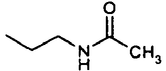 | 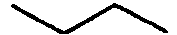 | 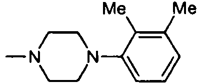 | 436 | I |
| 447 | 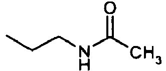 | 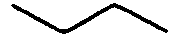 | 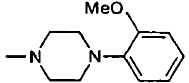 | 438 | I |
| 448 | 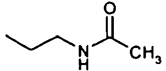 | 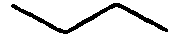 | 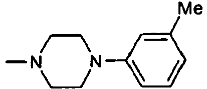 | 422 | I |
| 449 | 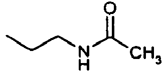 | 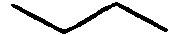 | 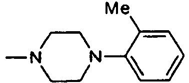 | 422 | I |
| 450 | 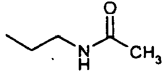 | 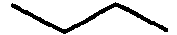 | 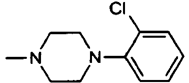 | 442 | I |
| 451 | 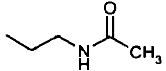 | 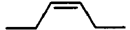 | 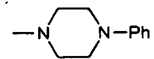 | 434 | I |
| 452 | 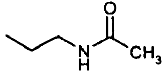 | 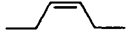 | 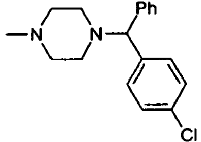 | 558 | I |
| 453 | 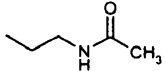 | 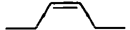 | 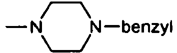 | 448 | I |
| 454 | 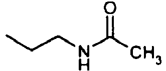 | 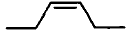 | 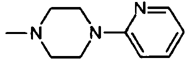 | 435 | I |
| 455 | 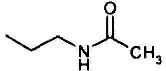 | 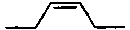 | 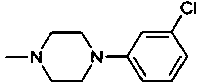 | 468 | I |
| 456 | 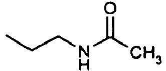 | 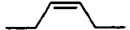 | 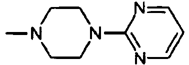 | 436 | I |
| 457 | 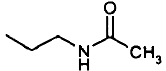 | 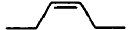 | 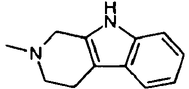 | 444 | I |
| 458 | 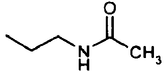 | 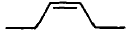 | 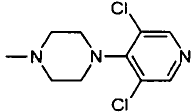 | 503 | I |
| 459 | 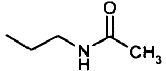 | 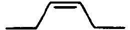 | 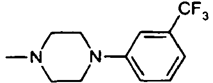 | 502 | I |
| 460 | 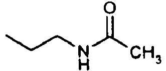 | 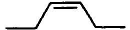 | 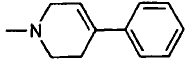 | 431 | I |
| 461 | 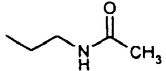 | 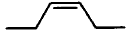 | 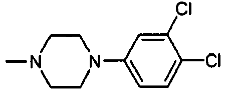 | 502 | I |
| 462 | 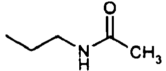 | 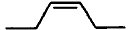 | 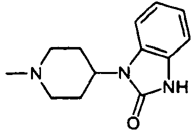 | 489 | I |
| 463 | 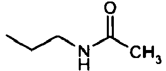 | 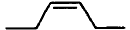 | 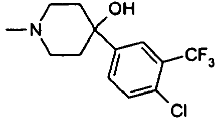 | 551 | I |
| 464 | 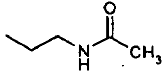 | 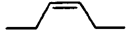 | 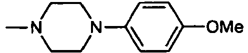 | 464 | I |
| 465 | 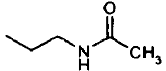 | 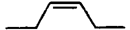 | 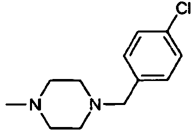 | 482 | I |
| 466 | 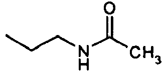 | 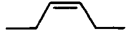 | 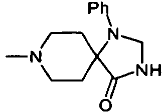 | 503 | I |
| 467 | 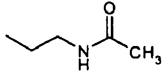 | 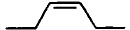 | 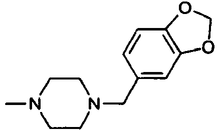 | 492 | I |
| 468 | 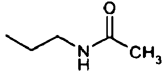 | 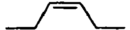 | 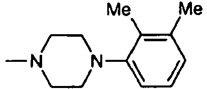 | 462 | I |
| 469 | 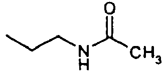 | 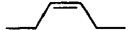 | 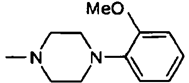 | 464 | I |
| 470 | 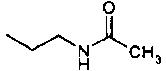 | 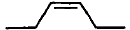 | 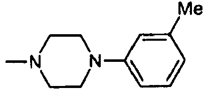 | 448 | I |
| 471 | 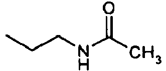 | 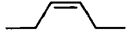 | 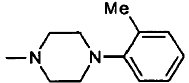 | 448 | I |
| 472 | 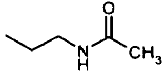 | 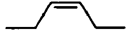 | 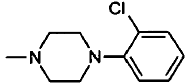 | 468 | I |
| 473 | 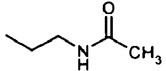 | 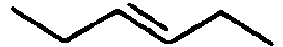 | 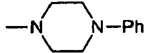 | 434 | I |
| 474 | 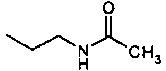 | 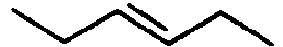 | 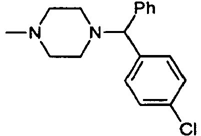 | 558 | I |
| 475 | 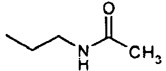 | 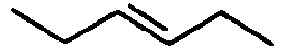 | 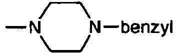 | 448 | I |
| 476 | 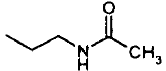 | 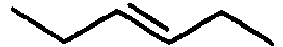 | 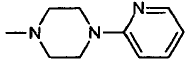 | 435 | I |
| 477 | 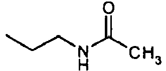 | 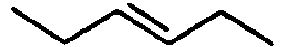 | 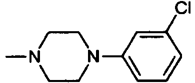 | 468 | I |
| 478 | 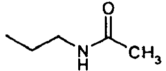 | 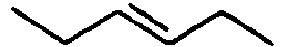 | 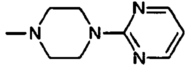 | 436 | I |
| 479 | 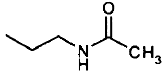 | 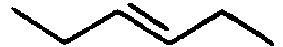 | 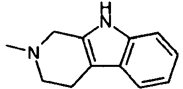 | 444 | I |
| 480 | 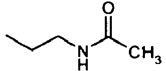 | 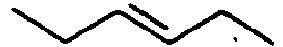 | 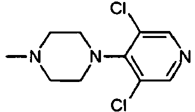 | 503 | I |
| 481 | 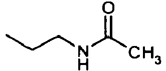 | 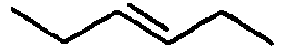 | 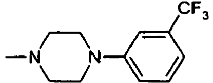 | 502 | I |
| 482 | 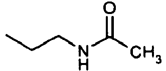 | 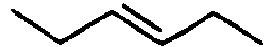 | 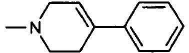 | 431 | I |
| 483 | 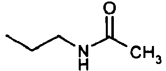 | 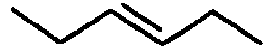 | 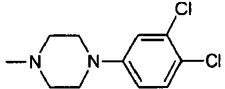 | 502 | I |
| 484 | 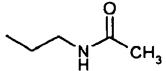 | 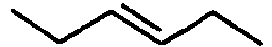 | 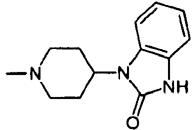 | 489 | I |
| 485 | 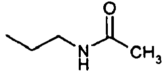 | 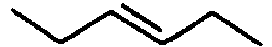 | 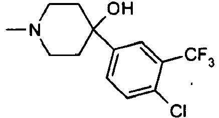 | 551 | I |
| 486 | 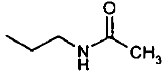 | 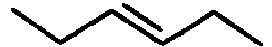 | 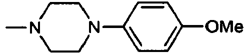 | 464 | I |
| 487 | 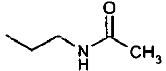 | 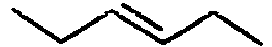 | 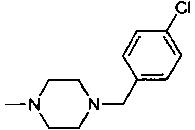 | 482 | I |
| 488 | 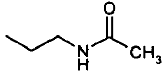 | 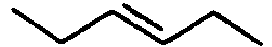 | 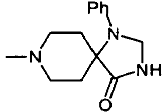 | 503 | I |
| 489 | 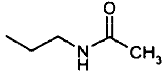 | 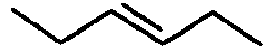 | 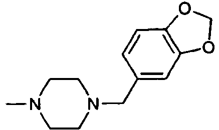 | 492 | I |
| 490 | 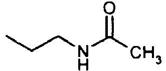 | 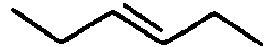 | 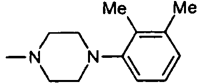 | 462 | I |
| 491 | 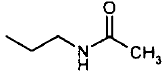 | 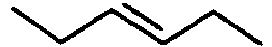 | 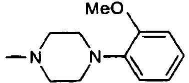 | 464 | I |
| 492 | 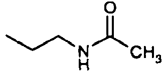 | 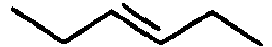 | 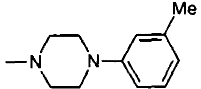 | 448 | I |
| 493 | 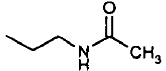 | 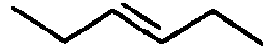 | 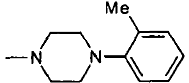 | 448 | I |
| 494 | 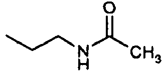 | 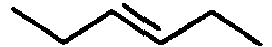 | 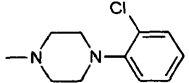 | 468 | I |
| 495 | 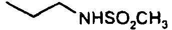 | 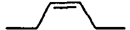 | 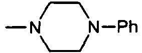 | 470 | I |
| 496 | 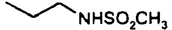 | 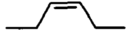 | 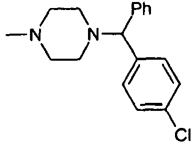 | 594 | I |
| 497 | 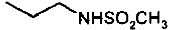 | 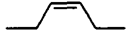 | 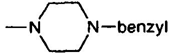 | 484 | I |
| 498 | 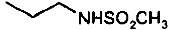 | 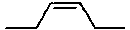 | 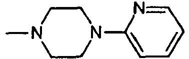 | 471 | I |
| 499 | 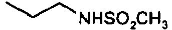 | 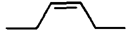 | 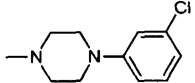 | 504 | I |
| 500 | 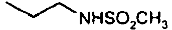 | 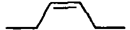 | 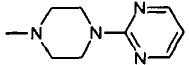 | 472 | I |
| 501 | 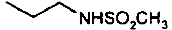 | 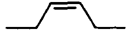 | 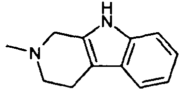 | 480 | I |
| 502 | 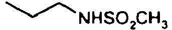 | 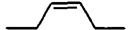 | 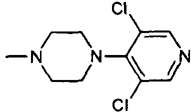 | 539 | I |
| 503 | 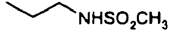 | 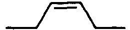 | 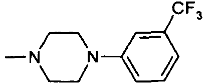 | 538 | I |
| 504 | 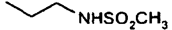 | 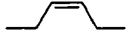 | 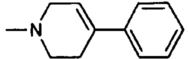 | 467 | I |
| 505 | 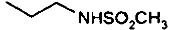 | 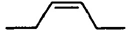 | 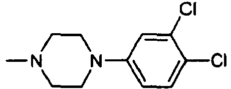 | 538 | I |
| 506 | 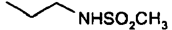 | 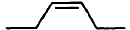 | 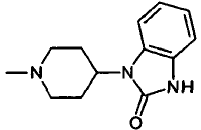 | 525 | I |
| 507 | 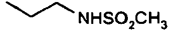 | 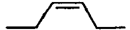 | 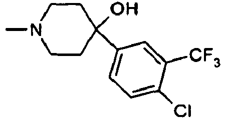 | 587 | I |
| 508 | 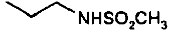 | 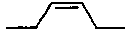 | 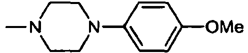 | 500 | I |
| 509 | 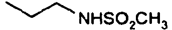 | 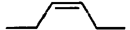 | 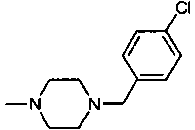 | 518 | I |
| 510 | 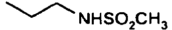 | 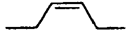 | 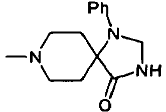 | 539 | I |
| 511 | 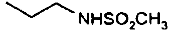 | 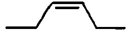 | 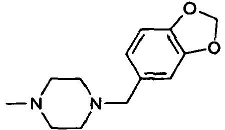 | 528 | I |
| 512 | 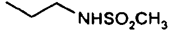 | 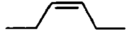 | 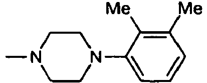 | 498 | I |
| 513 | 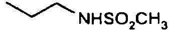 | 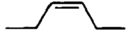 | 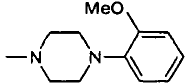 | 500 | I |
| 514 | 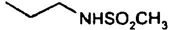 | 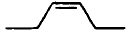 | 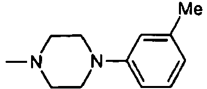 | 484 | I |
| 515 | 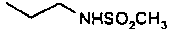 | 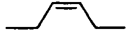 | 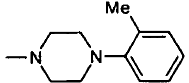 | 484 | I |
| 516 | 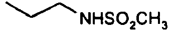 | 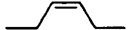 | 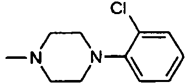 | 504 | I |
| 517 | 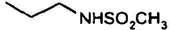 | 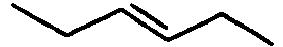 | 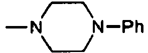 | 470 | I |
| 518 | 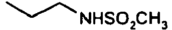 | 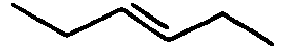 | 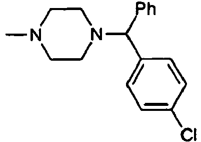 | 594 | I |
| 519 | 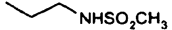 | 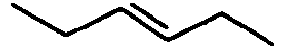 | 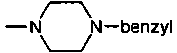 | 484 | I |
| 520 | 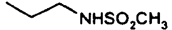 | 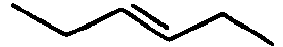 | 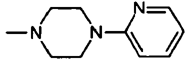 | 471 | I |
| 521 | 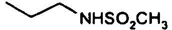 | 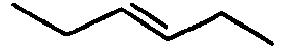 | 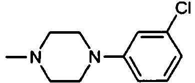 | 504 | I |
| 522 | 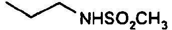 | 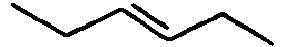 | 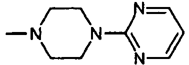 | 472 | I |
| 523 | 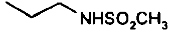 | 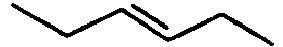 | 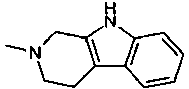 | 480 | I |
| 524 | 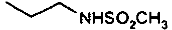 | 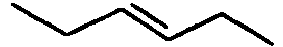 | 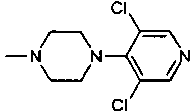 | 539 | I |
| 525 | 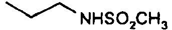 | 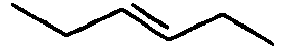 | 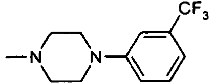 | 538 | I |
| 526 | 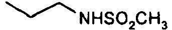 | 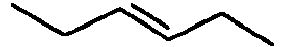 | 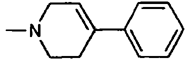 | 467 | I |
| 527 | 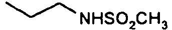 | 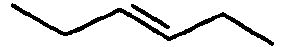 | 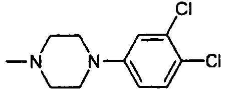 | 538 | I |
| 528 | 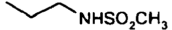 | 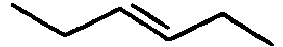 | 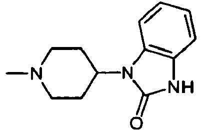 | 525 | I |
| 529 | 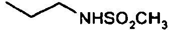 | 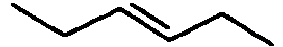 | 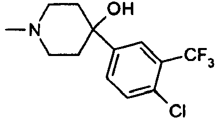 | 587 | I |
| 530 | 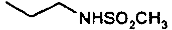 | 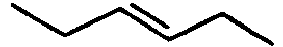 | 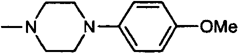 | 500 | I |
| 531 | 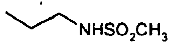 | 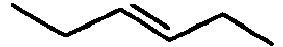 | 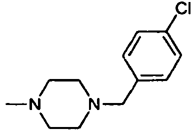 | 518 | I |
| 532 | 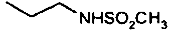 | 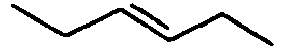 | 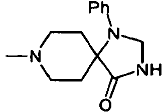 | 539 | I |
| 533 | 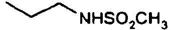 | 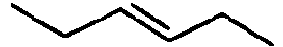 | 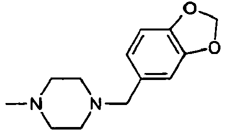 | 528 | I |
| 534 | 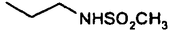 | 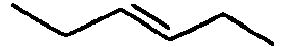 | 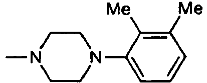 | 498 | I |
| 535 | 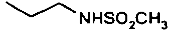 | 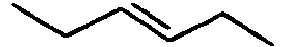 | 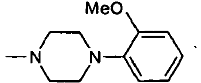 | 500 | I |
| 536 | 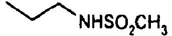 | 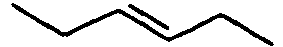 | 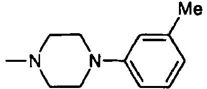 | 484 | I |
| 537 | 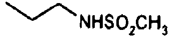 | 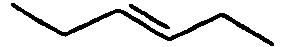 | 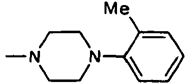 | 484 | I |
| 538 | 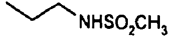 | 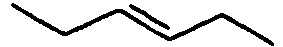 | 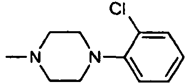 | 504 | I |

| | | | | | |
|---|---|---|---|---|---|
| R*: reference example I*: example according to the invention | | | | | |

The biological profile of the compounds object of this invention, was assessed by evaluating their activity at the 5-HT_{1A}, 5-HT_{2A} and D₄ receptors, according to the methods below described.

### Receptor Binding studies

Receptor binding studies were carried out to determine the affinity of the compounds for 5-HT_{1A}, 5-HT_{2A} and D₄ receptors

### 5-HT_{1A} Radioligand Receptor Binding Assay

Membranes from CHO cells, expressing 5-HT_{1A} human receptors were suspended in incubation buffer.

### Binding assay:

Binding assays were performed in MultiProbe 204 pipetting system (Packard), according to a predetermined mapping, consistent with the software Screen. The compounds were tested in singlicate at one concentration (10⁻⁷ M) in a total volume of 1000 µl. 980 µl of diluted membranes, 10 µl DMSO or unlabelled ligand and 10 µl of [³H]-8-OH-DPAT (0.6-0.7 nM) were incubated for 60 min at 27 °C. The reaction was stopped by rapid filtration through Tomtec cell Harvester (48 wells) using Filtermat B (presoaked in 0.1% PEI) filters. Filters were washed with ice-cold 50 mM Tris-HCl (pH 7.4) buffer (9 x 700 µl), dryed, covered with MeltiLex B/HS scintillator sheets (Wallac) and heated at 80-90 °C for about 10 min, transferred into plastic sample bags (Wallac), sealed and put into 1024 Beta Plate scintillation counter (Wallac). Non specific binding was determined in the presence of 5-HT (10⁻⁵ M).

### Data analysis:

The specific radioligand binding to the receptor was defined by the difference between total binding and non specific binding, determined in the presence of an excess of unlabelled ligand. Results were expressed as percentage of control specific binding obtained in the presence of the compounds.

The affinity values (IC₅₀) for the compounds were obtained by a non linear least squares regression analysis on the basis of a one binding site model.

### 5-HT1_{A} Functional assay (cAMP)

CHO/5-HT1_{A} cells were random seeded at a density of about 200,000/well in 24 well plates the day prior to the experiment. On the day of the experiment, cells were pretreated for 15 min at 37 °C with 500 µM isobuthylmethylxantine (IBMX) dissolved in culture medium without serum. Wells were then divided in different groups in duplicate as follows: control, 10 µM FSK, 10 µM FSK + 1 µM 5-HT as positive standard and 10 µM FSK + 10 µM of the different compound under evaluation. Sample solutions were added and incubated for additional 15 min at 37 °C. After incubation, medium was aspirated and the reaction stopped by adding 200 µl of lysis buffer. Plates were shaken for 5 min, then the lysate was removed and samples were stored at 4 °C until the day of the assay. For the cAMP evaluation, samples were properly diluted and the cAMP content was measured by an enzymeimmunoassay system.

### Data analysis:

Results are expressed as % inhibition of the cAMP accumulation induced by 10 µM FSK.

### D₄ Radioligand Receptor Binding Assay

Membranes from CHO cells, expressing D₄ human receptors were suspended in incubation buffer.

### Binding assay:

Binding assays were performed in MultiProbe 204 pipetting system (Packard), according to a predetermined mapping, consistent with the software Screen. The compounds were tested in singlicate at one concentration (10⁻⁷ M) in a total volume of 1000 µl (980 µl of diluted membranes, 10 µl DMSO or unlabelled ligand and 10 µl of [³H] YM-09151-2 (0.15-0.25 nM). After incubation for 120 min at 27 °C, the reaction was stopped by rapid filtration through Tomtec cell Harvester (48 wells) using Filtermat B (presoaked in 0.1% PEI) filters. Filters were washed with ice-cold 50 mM Tris-HCl (pH 7.4) buffer (9 x 700 µl), dryed, covered with MeltiLex B/HS (Wallac) scintillator sheets and heated in oven at 80-90 °C for about 10 min, transferred into plastic sample bags (Wallac), sealed and put into 1024 Beta Plate scintillation counter (Wallac). Non specific binding was determined in the presence of clozapine dissolved in DMSO to a final concentration of 10⁻⁵ M.

### Data analysis:

The specific radioligand binding to the receptor was defined by the difference between total binding and non specific binding, determined in the presence of an excess of unlabelled ligand. Results were expressed as percentage of control specific binding obtained in the presence of the compounds.

### 5HT_{2A} Radioligand Receptor Binding Assay

### Tissue preparation:

Rats (male Sprague-Dawley, 200-250 g) were used. Cerebral frontal cortex was homogenized in 10 volumes of ice cold 0.32 M sucrose in 5 mM Tris-HCl (pH 74) buffer. After centrifugation of the homogenate (1,000 x g for 10 min) the supernatant was then recentrifuged at 48,000x g for 15 min.The resulting pellet was gently homogenized in an equal volume of 50mM Tris-HCl buffer (pH 7.4) and incubated at 37° C for 10 minutes. Membranes were then collected by centrifugation as above described and finally resuspended in 10 volumes of 50mM Tris-HCl buffer (pH 7.4).

### Binding assay:

For displacement experiments membranes (980 µl) were diluted in 50 mM Tris-HCl buffer (pH 7.4) to a final concentration of 1:100 (w/v); the tissue suspension was then incubated at 37 °C for 10 min in a final volume of 1 ml in the presence of 0.5 nM [³H]-Ketanserin. Non specific binding was determined by incubating similar samples with unlabelled methysergide (100 µM). After incubation, samples prepared in a 24 wells cell culture cluster (Costar) were rapidly filtered by Inotech cell harvester (IH 201 filters). The filters were washed three times with 2 ml ice-cold Tris-HCl buffer and placed in polyethylene vials, then 4 ml of Filter Count scintillation cocktail (Packard) were added. The radioactivity present was counted by liquid scintillation spectrometry.

### Data analysis:

The affinity values (IC₅₀) for the compounds were obtained by a non linear least squares regression analysis on the basis of a one binding site model.

### 5HT_{2A} Functional assay (PI turnover)

### Tissue preparation:

Cross-chopped miniprisms (350 x 350 µm) were prepared from mouse whole cerebral cortices and incubated for 60 min at 37 °C in Krebs-Henseleit buffer containing 2 g/l glucose.

### Functional assay:

Cerebral cortex miniprisms were distribuited in vials and incubated for 30 min with approximately 170 nM [³H]-myoinositol (10-20 Ci/mmol) and 10 mM lithium chloride. Samples were divided in different groups in triplicate: control, 100 µM 5-HT, 10 and 30 µM flibanserin + 100 µM 5-HT, as standards, and 10 µM of the different compound under investigation + 100 µM 5-HT. When 5-HT was added the incubation continued for 45 min. Compounds under investigastion and flibanserin were added 10 min before dispensing 5-HT. Incubation was terminated by the addition of 940 µl chloroform-methanol (1: 2 v/v). Further aliquots of chloroform (310 µl) and water (310 µl) were added and labeled inositol phosphates (I Ps) were extracted from the aqueous phase by ion exchange chromatography using Dowex resin in the formiate form. After addition of 10 ml of PicoFluor 40 scintillation cocktail (Packard), the radioactivity present in an aliquot (400 µl) of the aqueous extract was counted by liquid scintillation spectrometry.

### Data analysis:

Results are expressed as % inhibition of the PI turnover accumulation induced by 100 µM 5-HT.

The following tables (Tables 4 to 6) collect the biological data at the said receptors of the new compounds.

**Table 4: % inhibition at 5-HT1_{A} and D₄ receptors**

| Comp. No. | 5-HT1_{A} receptor binding assay % inhib. (10⁻⁷ M) | D₄ receptor binding assay % inhib. (10⁻⁷ M) | Comp. No. | 5-HT1_{A} receptor binding assay % inhib. (10⁻⁷ | D₄ receptor binding assay % inhib. (10⁻⁷ M) |
|---|---|---|---|---|---|
| 1 | 56 | 38 | 78 | 69 | 44 |
| 5 | 92 | 54 | 79 | 86 | 58 |
| 7 | 77 | 91 | 81 | 55 | 78 |
| 9 | 93 | 32 | 83 | 89 | 39 |
| 10 | 60 | 47 | 84 | 52 | 42 |
| 11 | 48 | 90 | 85 | 77 | 79 |
| 19 | 69 | 32 | 92 | 94 | 55 |
| 20 | 50 | 60 | 93 | 94 | 62 |
| 23 | 73 | 48 | 94 | 88 | 72 |
| 24 | 90 | 67 | 95 | 85 | 64 |
| 25 | 48 | 44 | 96 | 92 | 72 |
| 26 | 70 | 94 | 107 | 55 | 58 |
| 28 | 89 | 35 | 118 | 80 | 36 |
| 29 | 57 | 83 | 145 | 85 | 42 |
| 30 | 44 | 90 | 149 | 88 | 35 |
| 37 | 90 | 54 | 150 | 57 | 52 |
| 38 | 92 | 78 | 158 | 95 | 72 |
| 39 | 36 | 42 | 159 | 85 | 50 |
| 43 | 104 | 55 | 164 | 96 | 41 |
| 45 | 100 | 82 | 169 | 85 | 58 |
| 56 | 63 | 71 | 177 | 98 | 39 |
| 59 | 75 | 51 | 182 | 62 | 45 |
| 60 | 93 | 82 | 183 | 96 | 62 |
| 62 | 73 | 96 | 187 | 94 | 36 |
| 64 | 92 | 43 | 188 | 78 | 78 |
| 65 | 52 | 74 | 189 | 54 | 99 |
| 66 | 65 | 99 | 197 | 77 | 43 |
| 73 | 91 | 58 | 215 | 98 | 48 |
| 74 | 92 | 62 | 216 | 92 | 44 |
| 75 | 67 | 54 | 219 | 89 | 37 |
| 224 | 98 | 51 | 332 | 78 | 85 |
| 226 | 71 | 32 | 333 | 99 | 80 |
| 228 | 95 | 33 | 335 | 95 | 55 |
| 229 | 67 | 34 | 336 | 90 | 81 |
| 241 | 69 | 32 | 348 | 99 | 51 |
| 254 | 85 | 34 | 349 | 73 | 31 |
| 255 | 58 | 33 | 361 | 86 | 46 |
| 256 | 59 | 51 | 364 | 77 | 36 |
| 263 | 92 | 42 | 365 | 89 | 63 |
| 265 | 55 | 78 | 367 | 61 | 90 |
| 280 | 93 | 38 | 369 | 96 | 59 |
| 282 | 53 | 77 | 370 | 93 | 60 |
| 285 | 96 | 32 | 371 | 59 | 95 |
| 286 | 65 | 87 | 378 | 77 | 35 |
| 293 | 89 | 32 | 382 | 88 | 39 |
| 297 | 96 | 34 | 395 | 86 | 32 |
| 303 | 71 | 70 | 399 | 82 | 32 |
| 310 | 85 | 49 | 404 | 95 | 34 |
| 311 | 59 | 60 | 412 | 93 | 39 |
| 314 | 93 | 82 | 416 | 96 | 39 |
| 316 | 72 | 94 | 446 | 92 | 31 |
| 318 | 92 | 36 | 501 | 70 | 63 |
| 319 | 94 | 70 | 504 | 93 | 34 |
| 327 | 78 | 74 | 505 | 78 | 58 |
| 330 | 97 | 84 | 527 | 73 | 36 |
| 331 | 100 | 92 | 535 | 97 | 39 |

**Table 5: 5-HT_{1A} agonist activity**

| Comp.d No. | **5-HT**_{1A} | |
|---|---|---|
| | receptor binding IC₅₀ (nM) | cAMP % inhib. |
| 5 | 13 | 63 |
| 9 | 9.9 | 48 |
| 37 | 16 | 44 |
| 60 | 6.2 | 65 |
| 64 | 12 | 52 |
| 73 | 13 | 45 |
| 83 | 15 | 64 |
| 158 | 13 | 48 |
| 164 | 4.2 | 73 |
| 168 | 5.0 | 71 |
| 177 | 3.3 | 76 |
| 183 | 7.2 | 66 |
| 187 | 8.2 | 44 |
| 202 | 1.7 | 72 |
| 206 | 2.1 | 80 |
| 215 | 0.85 | 83 |
| 217 | 5.2 | 68 |
| 219 | 15 | 61 |
| 228 | 6.0 | 82 |
| 254 | 7.4 | 66 |
| 263 | 8.8 | 63 |
| 284 | 4.9 | 82 |
| 285 | 5.2 | 47 |
| 296 | 8.2 | 82 |
| 297 | 7.9 | 74 |
| 301 | 2.6 | 83 |
| 310 | 15 | 63 |
| 314 | 7.1 | 44 |
| 318 | 3.1 | 61 |
| 330 | 7.9 | 62 |
| 333 | 16 | 67 |
| 335 | 3.5 | 69 |
| 347 | 13 | 65 |
| 348 | 3.5 | 57 |
| 352 | 4.1 | 79 |
| 365 | 15 | 82 |
| 369 | 3.5 | 84 |
| 370 | 4.4 | 52 |
| 381 | 44 | 79 |
| 382 | 11 | 64 |
| 386 | 6.7 | 82 |
| 403 | 5.5 | 84 |
| 404 | 2.1 | 60 |
| 415 | 14 | 82 |
| 416 | 7.9 | 77 |
| 420 | 1.8 | 87 |
| 421 | 0.66 | 56 |
| 446 | 7.3 | 81 |
| 459 | 8.8 | 86 |
| 468 | 3.1 | 66 |
| 472 | 3.1 | 67 |
| 481 | 11 | 82 |
| 499 | 5.0 | 74 |
| 503 | 2.8 | 87 |
| 504 | 3.6 | 50 |
| 512 | 0.59 | 68 |
| 514 | 7.9 | 67 |
| 520 | 8.1 | 70 |
| 521 | 0.61 | 67 |
| 525 | 1.5 | 87 |
| 536 | 9.5 | 55 |

**Table 6: 5-HT_{2A} antagonist activity**

| Comp.d No. | **5-HT**_{2A} | |
|---|---|---|
| | receptor binding IC₅₀ (nM) | PI turnover % inhib. |
| 9 | 16 | 45 |
| 73 | 0.90 | 42 |
| 83 | 43 | 86 |
| 168 | 46 | 22.00 |
| 177 | 7.7 | 39 |
| 183 | 27 | 12.00 |
| 206 | 17 | 90 |
| 215 | 3.2 | 83 |
| 254 | 65 | 64 |
| 514 | 74 | 41 |
| 521 | 30 | 48 |

## Claims

1. Compounds of general formula (I) 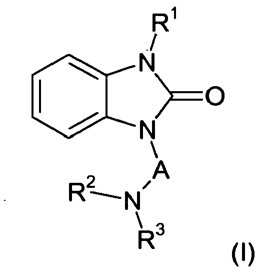 wherein
R¹ denotes C₁-C₆-alkyl, being substituted by a group selected from C₁-C₆-alkoxy, -OCONHC₁-C₆-alkyl, -NHSO₂C₁-C₆-alkyl and -NHCOC₁-C₆-alkyl;
R² and R³ together with the nitrogen form a saturated or unsaturated 5- or 6-membered heterocyclic ring which may contain nitrogen or oxygen as an additional heteroatom, whilst the heterocyclic ring is substituted by a group selected from phenyl, benzyl and diphenylmethyl, said group being optionally mono- or di-substituted by one or two groups selected from CF₃, C₁-C₄-alkyl, C₁-C₄-alkoxy, phenyl, benzyl, halogen and OH, or
R² and R³ together with the nitrogen form a saturated or unsaturated 5- or 6-membered heterocyclic ring which may contain nitrogen or oxygen as an additional heteroatom, said heterocyclic ring being linked via a single bond, a methylene-bridge or spiro-connected to another saturated or unsaturated heterocyclic group containing one or two heteroatoms selected from oxygen and nitrogen, said heterocyclic group being optionally mono- or di-substituted by a group selected from CF₃, C₁-C₄-alkyl, C₁-C₄-alkoxy, phenyl, benzyl, halogen, =O and OH, or
R² and R³ together with the nitrogen form a saturated or unsaturated bi- or tricyclic heterocyclic ring-system which may contain nitrogen or oxygen as an additional heteroatom, said heterocyclic ring-system being optionally substituted by a group selected from CF₃, C₁-C₄-alkyl, C₁-C₄-alkoxy, phenyl, benzyl, halogen, =O and OH;
A denotes C₁-C₆-alkylen, C₂-C₆-alkenylen, or C₂-C₆-alkynylen,
or a pharmaceutically acceptable salt thereof.

2. Compounds of formula (I) according to claim 1, wherein
R' denotes C₁-C₄-alkyl, being substituted by a group selected from C₁-C₄-alkoxy, -OCONHC₁-C₄-alkyl, -NHSO₂C₁-C₄-alkyl and -NHCOC₁-C₄-alkyl;
R² and R³ together with the nitrogen form a saturated or unsaturated 5- or 6-membered heterocyclic ring which may contain nitrogen as an additional heteroatom, whilst the heterocyclic ring is substituted by a group selected from phenyl, benzyl, diphenylmethyl, pyridinyl, pyrimidinyl, benzimidazolonyl and 3,4-methylendioxibenzyl, said group being optionally mono- or di-substituted by a group selected from CF₃, C₁-C₄-alkyl, C₁-C₄-alkoxy, halogen and OH;
A denotes C₁-C₄-alkylen or C₂-C₄-alkenylen,
or a pharmaceutically acceptable salt thereof.

3. Compounds of formula (I) according to claim 1 or 2, wherein
R¹ denotes ethyl, being substituted by a group selected from OCH₃, OCH₂CH₃, - OCONHCH₃, -OCONHCH₂CH₃, -NHSO₂CH₃, -NHSO₂CH₂CH₃, -NHCOCH₃, and -NHCOCH₂CH₃;
R² and R³ together with the nitrogen form a 6 membered saturated or unsaturated heterocyclic ring which may contain nitrogen as an additional heteroatom, whilst the heterocyclic ring is substituted by a group selected from phenyl, pyridinyl, pyrimidinyl, benzimidizalonyl, and substituted phenyl being mono- or di-substituted by a group selected from CF₃, CH₃, OCH₃, F and Cl;
A denotes C₁-C₄-alkylen or C₂-C₄-alkenylen,
or a pharmaceutically acceptable salt thereof.

4. Compounds of formula (I) according to claim 1, 2 or 3, wherein
R¹ denotes ethyl, being substituted by a group selected from OCH₃, -OCONHCH₂CH₃, -NHSO₂CH₃ and -NHCOCH₃;
R² and R³ together with the nitrogen form a 6 membered saturated or unsaturated heterocyclic ring which may contain nitrogen as an additional heteroatom, whilst the heterocyclic ring is substituted by a group selected from pyridinyl, phenyl and substituted phenyl being mono- or di-substituted by a group selected from CF₃, CH₃, OCH₃, F and Cl;
A denotes ethylen, propylen, butylen or butenylen,
or a pharmaceutically acceptable salt thereof.

5. Compounds of formula (I) according to one of claims 1 to 4, wherein
R¹ denotes ethyl, being substituted by a group selected from OCH₃,-OCONHCH₂CH₃, -NHSO₂CH₃ and -NHCOCH₃;
R² and R³ together with the nitrogen form a ring selected from the group consisting of piperazin, piperidin and tetrahydropyridin, which is substituted by a group selected from pyridinyl, phenyl and substituted phenyl being mono- or di-substituted by a group selected from CF₃, CH₃ and Cl;
A denotes ethylen, butylen or butenylen,
or a pharmaceutically acceptable salt thereof.

6. Compounds of formula (I) in claim 1, wherein
R¹ denotes ethyl, being substituted by a group selected from OCH₃, - OCONHCH₂CH₃ and -NHSO₂CH₃;
R² and R³ together with the nitrogen form a piperazine-ring, being substituted by a group selected from trifluoromethylphenyl, methylphenyl, dimethylphenyl and chlorophenyl;
A denotes ethylen, butylen or butenylen,
or a pharmaceutically acceptable salt thereof.

7. A pharmaceutical composition comprising one or more compounds of formula (I) according to one of claims 1 to 6.

8. Use of a compound of formula (I) according to one of claims 1 to 6 for the preparation of a pharmaceutical composition for the treatment of anxiety disorders and affective disorders.

9. Use according to claim 8 for the preparation of a pharmaceutical composition for the treatment of a desease selected from depression, psychosis, schizophrenia, eating disorders, sexual disorders, Parkinson, stroke and traumatic brain injury.

## Patentansprüche

1. Verbindungen der allgemeinen Formel (I) 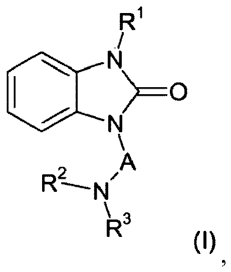 worin
R¹ C₁-C₆-Alkyl bezeichnet, das durch eine Gruppe substituiert ist, die aus C₁-C₆-Alkoxy, -OCONHC₁-C₆-Alkyl, -NHSO₂C₁-C₆-Alkyl und -NHCOC₁-C₆-Alkyl ausgewählt ist;
R² und R³ zusammen mit dem Stickstoff einen gesättigten oder ungesättigten heterocyclischen 5- oder 6-Ring bilden, der Stickstoff oder Sauerstoff als zusätzliches Heteroatom enthalten kann, während der heterocyclische Ring durch eine Gruppe substituiert ist, die aus Phenyl, Benzyl und Diphenylmethyl ausgewählt ist, wobei die genannte Gruppe optional durch eine oder zwei Gruppen mono- oder disubstituiert ist, die aus CF₃, C₁-C₄-Alkyl, C₁-C₄-Alkoxy, Phenyl, Benzyl, Halogen und OH ausgewählt ist/sind, oder
R² und R³ zusammen mit dem Stickstoff einen gesättigten oder ungesättigten heterocyclischen 5- oder 6-Ring bilden, der Stickstoff oder Sauerstoff als zusätzliches Heteroatom enthalten kann, wobei der genannte heterocyclische Ring über eine Einfachbindung, eine Methylen-Brücke oder Spiro-verbunden zu einer anderen gesättigten oder ungesättigten heterocyclischen Gruppe verknüpft ist, die ein oder zwei Heteroatome enthält, das/die aus Sauerstoff und Stickstoff ausgewählt ist/sind, wobei die genannte heterocyclische Gruppe optional durch eine Gruppe mono- oder disubstituiert ist, die aus CF₃, C₁-C₄-Alkyl, C₁-C₄-Alkoxy, Phenyl, Benzyl, Halogen, =O und OH ausgewählt ist, oder
R² und R³ zusammen mit dem Stickstoff ein gesättigtes oder ungesättigtes bi- oder tricyclisches heterocyclisches Ringsystem bilden, das Stickstoff oder Sauerstoff als zusätzliches Heteroatom enthalten kann, wobei das genannte heterocyclische Ringsystem optional durch eine Gruppe substituiert ist, die aus CF₃, C₁-C₄-Alkyl, C₁-C₄-Alkoxy, Phenyl, Benzyl, Halogen, =O und OH ausgewählt ist;
A C₁-C₆-Alkylen, C₂-C₆-Alkenylen oder C₂-C₆-Alkinylen bezeichnet,
oder ein pharmazeutisch verträgliches Salz davon.

2. Verbindungen der Formel (I) nach Anspruch 1, worin
R¹ C₁-C₄-Alkyl bezeichnet, das durch eine Gruppe substituiert ist, die aus C₁-C₄-Alkoxy, -OCONHC₁-C₄-Alkyl, -NHSO₂C₁-C₄-Alkyl und -NHCOC₁-C₄-Alkyl ausgewählt ist;
R² und R³ zusammen mit dem Stickstoff einen gesättigten oder ungesättigten heterocyclischen 5- oder 6-Ring bilden, der Stickstoff als zusätzliches Heteroatom enthalten kann, während der heterocyclische Ring durch eine Gruppe substituiert ist, die aus Phenyl, Benzyl, Diphenylmethyl, Pyridinyl, Pyrimidinyl, Benzimidazolonyl und 3,4-Methylendioxybenzyl ausgewählt ist, wobei die genannte Gruppe optional durch eine Gruppe mono- oder disubstituiert ist, die aus CF₃, C₁-C₄-Alkyl, C₁-C₄-Alkoxy, Halogen und OH ausgewählt ist;
A C₁-C₄-Alkylen oder C₂-C₄-Alkenylen bezeichnet,
oder ein pharmazeutisch verträgliches Salz davon.

3. Verbindungen der Formel (I) nach Anspruch 1 oder 2, worin
R¹ Ethyl bezeichnet, das durch eine Gruppe substituiert ist, die aus OCH₃, OCH₂CH₃, -OCONHCH₃, -OCONHCH₂CH₃, -NHSO₂CH₃, -NHSO₂CH₂CH₃, -NHCOCH₃ und -NHCOCH₂CH₃ ausgewählt ist;
R² und R³ zusammen mit dem Stickstoff einen gesättigten oder ungesättigten heterocyclischen 6-Ring bilden, der Stickstoff als zusätzliches Heteroatom enthalten kann, während der heterocyclische Ring durch eine Gruppe substituiert ist, die aus Phenyl, Pyridinyl, Pyrimidinyl, Benzimidazolonyl und substituiertem Phenyl ausgewählt ist, das durch eine Gruppe mono- oder disubstituiert ist, die aus CF₃, CH₃, OCH₃, F und Cl ausgewählt ist;
A C₁-C₄-Alkylen oder C₂-C₄-Alkenylen bezeichnet,
oder ein pharmazeutisch verträgliches Salz davon.

4. Verbindungen der Formel (I) nach Anspruch 1, 2 oder 3, worin
R¹ Ethyl bezeichnet, das durch eine Gruppe substituiert ist, die aus OCH₃, -OCONHCH₂CH₃, -NHSO₂CH₃ und -NHCOCH₃ ausgewählt ist;
R² und R³ zusammen mit dem Stickstoff einen gesättigten oder ungesättigten heterocyclischen 6-Ring bilden, der Stickstoff als zusätzliches Heteroatom enthalten kann, während der heterocyclische Ring durch eine Gruppe substituiert ist, die aus Pyridinyl, Phenyl und substituiertem Phenyl ausgewählt ist, das durch eine Gruppe mono- oder disubstituiert ist, die aus CF₃, CH₃, OCH₃, F und Cl ausgewählt ist;
A Ethylen, Propylen, Butylen oder Butenylen bezeichnet,
oder ein pharmazeutisch verträgliches Salz davon.

5. Verbindungen der Formel (I) nach einem der Ansprüche 1 bis 4, worin
R¹ Ethyl bezeichnet, das durch eine Gruppe substituiert ist, die aus OCH₃, -OCONHCH₂CH₃, -NHSO₂CH₃ und -NHCOCH₃ ausgewählt ist;
R² und R³ zusammen mit dem Stickstoff einen Ring bilden, der aus der Gruppe ausgewählt ist, die aus Piperazin, Piperidin und Tetrahydropyridin besteht, das durch eine Gruppe substituiert ist, die aus Pyridinyl, Phenyl und substituiertem Phenyl ausgewählt ist, das durch eine Gruppe mono- oder disubstituiert ist, die aus CF₃, CH₃ und Cl ausgewählt ist;
A Ethylen, Butylen oder Butenylen bezeichnet,
oder ein pharmazeutisch verträgliches Salz davon.

6. Verbindungen der Formel (I) nach Anspruch 1, worin
R¹ Ethyl bezeichnet, das durch eine Gruppe substituiert ist, die aus OCH₃, -OCONHCH₂CH₃ und -NHSO₂CH₃ ausgewählt ist;
R² und R³ zusammen mit dem Stickstoff einen Piperazinring bilden, der durch eine Gruppe substituiert ist, die aus Trifluormethylphenyl, Methylphenyl, Dimethylphenyl und Chlorphenyl ausgewählt ist;
A Ethylen, Butylen oder Butenylen bezeichnet,
oder ein pharmazeutisch verträgliches Salz davon.

7. Pharmazeutische Zusammensetzung, die eine oder mehrere Verbindungen der Formel (I) nach einem der Ansprüche 1 bis 6 umfasst.

8. Verwendung einer Verbindung der Formel (I) nach einem der Ansprüche 1 bis 6 für die Herstellung einer pharmazeutischen Zusammensetzung für die Behandlung von Angststörungen und affektiven Störungen.

9. Verwendung nach Anspruch 8 für die Herstellung einer pharmazeutischen Zusammensetzung für die Behandlung einer Erkrankung, die aus Folgenden ausgewählt ist: Depression, Psychose, Schizophrenie, Essstörungen, sexuellen Störungen, Parkinson-Syndrom, Schlaganfall und traumatischer Hirnverletzung.

## Revendications

1. Composés de formule générale (I) 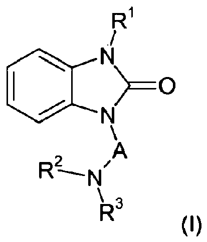 dans laquelle
R¹ désigne alkyle en C₁-C₆, substitué par un groupement choisi parmi alcoxy en C₁-C₆, -OCONHC₁-C₆-alkyle, -NHSO₂C₁-C₆-alkyle et -NHCOC₁-C₆-alkyle ;
R² et R³ conjointement avec l'azote forment un cycle hétérocyclique saturé ou insaturé à 5 ou 6 chaînons, qui peut contenir un azote ou un oxygène en tant qu'hétéroatome supplémentaire, tandis que le cycle hétérocyclique est substitué par un groupement choisi parmi phényle, benzyle et diphénylméthyle, ledit groupement étant éventuellement mono- ou disubstitué par un ou deux groupements choisis parmi CF₃, alkyle en C₁-C₄, alcoxy en C₁-C₄, phényle, benzyle, halogène et OH, ou
R² et R³ conjointement avec l'azote forment un cycle hétérocyclique saturé ou insaturé à 5 ou 6 chaînons, qui peut contenir un azote ou un oxygène en tant qu'hétéroatome supplémentaire, ledit cycle hétérocyclique étant lié par l'intermédiaire d'une simple liaison d'un pont méthylène ou relié au niveau d'un spiro à un autre groupement hétérocyclique saturé ou insaturé contenant un ou deux hétéroatomes choisis parmi oxygène et azote, ledit groupement hétérocyclique étant éventuellement mono- ou disubstitué par un ou deux groupements choisis parmi CF₃, alkyle en C₁-C₄, alcoxy en C₁-C₄, phényle, benzyle, halogène, =O et OH, ou
R² et R³ conjointement avec l'azote forment un système de cycle hétérocyclique bi- ou tricyclique saturé ou insaturé, qui peut contenir un azote ou un oxygène en tant qu'hétéroatome supplémentaire, ledit système de cycle hétérocyclique étant éventuellement substitué par un groupement choisi parmi CF₃, alkyle en C₁-C₄, alcoxy en C₁-C₄, phényle, benzyle, halogène, =O et OH ;
A désigne C₁-C₆-alkylène, C₂-C₆-alcénylène ou C₂-C₆-alcynylène,
ou un sel pharmaceutiquement acceptable de ceux-ci.

2. Composés de formule (I) selon la revendication 1, dans laquelle
R¹ désigne alkyle en C₁-C₄, substitué par un groupement choisi parmi alcoxy en C₁-C₄, -OCONHC₁-C₄-alkyle, -NHSO₂C₁-C₄-alkyle et -NHCOC₁-C₄-alkyle ;
R² et R³ conjointement avec l'azote forment un cycle hétérocyclique saturé ou insaturé à 5 ou 6 chaînons, qui peut contenir un azote en tant qu'hétéroatome supplémentaire, tandis que le cycle hétérocyclique est substitué par un groupement choisi parmi phényle, benzyle, diphénylméthyle, pyridinyle, pyrimidinyle, benzimidazolonyle et 3,4-méthylènedioxybenzyle, ledit groupement étant éventuellement mono- ou disubstitué par un groupement choisi parmi CF₃, alkyle en C₁-C₄, alcoxy en C₁-C₄, halogène et OH,
A désigne C₁-C₄-alkylène ou C₂-C₄-alcénylène,
ou un sel pharmaceutiquement acceptable de ceux-ci.

3. Composés de formule (I) selon la revendication 1 ou 2, dans laquelle
R¹ désigne éthyle, substitué par un groupement choisi parmi OCH₃, OCH₂CH₃, -OCONHCH₃, -OCONHCH₂CH₃, -NHSO₂CH₃, -NHSO₂ CH₂CH₃, -NHCOCH₃ et -NHCOCH₂CH₃ ;
R² et R³ conjointement avec l'azote forment un cycle hétérocyclique saturé ou insaturé à 6 chaînons, qui peut contenir un azote en tant qu'hétéroatome supplémentaire, tandis que le cycle hétérocyclique est substitué par un groupement choisi parmi phényle, pyridinyle, pyrimidinyle, benzimidizalonyle et phényle substitué étant mono- ou disubstitué par un groupement choisi parmi CF₃, CH₃, OCH₃, F et Cl ;
A désigne C₁-C₄-alkylène ou C₂-C₄-alcénylène,
ou un sel pharmaceutiquement acceptable de ceux-ci.

4. Composés de formule (I) selon la revendication 1, 2 ou 3, dans laquelle
R¹ désigne éthyle, substitué par un groupement choisi parmi OCH₃, -OCONHCH₂CH₃, -NHSO₂CH₃ et -NHCOCH₃ ;
R² et R³ conjointement avec l'azote forment un cycle hétérocyclique saturé ou insaturé à 6 chaînons, qui peut contenir un azote en tant qu'hétéroatome supplémentaire, tandis que le cycle hétérocyclique est substitué par un groupement choisi parmi pyridinyle, phényle et phényle substitué étant mono- ou disubstitué par un groupement choisi parmi CF₃, CH₃, OCH₃, F et Cl ;
A désigne éthylène, propylène, butylène ou buténylène,
ou un sel pharmaceutiquement acceptable de ceux-ci.

5. Composés de formule (I) selon l'une des revendications 1 à 4, dans laquelle
R¹ désigne éthyle, substitué par un groupement choisi parmi OCH₃, -OCONHCH₂CH₃, -NHSO₂CH₃ et -NHCOCH₃ ;
R² et R³ conjointement avec l'azote forment un cycle choisi dans le groupe constitué de pipérazine, pipéridine et tétrahydropyridine qui est substitué par un groupement choisi parmi pyridinyle, phényle et phényle substitué étant mono- ou disubstitué par un groupement choisi parmi CF₃, CH₃ et Cl;
A désigne éthylène, butylène ou buténylène,
ou un sel pharmaceutiquement acceptable de ceux-ci.

6. Composés de formule (I) selon la revendication 1, dans laquelle
R¹ désigne éthyle, substitué par un groupement choisi parmi OCH₃, -OCONHCH₂CH₃ et -NHSO₂CH₃ ;
R² et R³ conjointement avec l'azote forment un cycle pipérazine substitué par un groupement choisi parmi trifluorométhylphényle, méthylphényle, diméthylphényle et chlorophényle ;
A désigne éthylène, butylène ou buténylène,
ou un sel pharmaceutiquement acceptable de ceux-ci.

7. Composition pharmaceutique comprenant un ou plusieurs composés de formule (I) selon l'une des revendications 1 à 6.

8. Utilisation d'un composé de formule (I) selon l'une des revendications 1 à 6, pour la préparation d'une composition pharmaceutique pour le traitement de troubles de l'anxiété et de troubles affectifs.

9. Utilisation selon la revendication 8, pour la préparation d'une composition pharmaceutique destinée au traitement d'une maladie choisie parmi la dépression, la psychose, la schizophrénie, les troubles du comportement alimentaire, les troubles sexuels, la maladie de Parkinson, l'accident vasculaire cérébral et les lésions cérébrales traumatiques.
